# EUROPEAN PATENT APPLICATION

(11) **EP 1 482 043 A1**
(43) Date of publication of application: **01.12.2004**
(21) Application number: 03707173.5
(22) Date of filing: 28.02.2003
(51) Int. Cl.: C12N 15/13, C12N 1/15, C12N 1/19, C12N 1/21, C12N 5/10, C07K 16/44, C12P 21/02, G01N 33/53

(54) **PROTEINS CAPABLE OF BINDING TO ENVIRONMENTAL HORMONES AND PROCESS FOR PRODUCING THE SAME**

(30) Priority: 01.03.2002 JP 2002055670
(71) Applicant: Japan Envirochemicals, Ltd., Osaka-shi, Osaka 541-0045 (JP)
(72) Inventor: OHKAWA, Hideo, Kobe-shi, Hyogo 651-1255 (JP); FUJIMOTO, Shigeru, c/o Japan EnviroChemicals, Ltd, Osaka-shi, Osaka 532-0024 (JP); GODA, Yasuhiro, c/o Japan EnviroChemicals, Ltd., Osaka-shi, Osaka 532-0024 (JP)
(74) Representative: von Kreisler Selting Werner
(86) International application number: PCT/JP2003/002312
(87) International publication number: WO 2003/074705

(57) **Abstract**

The present invention provides a protein capable of binding to an environmental hormone, which protein is supplemented with useful properties for measuring, quantifying and concentrating environmental hormones, such as high sensitivity, low cross-reactivity, unlikelihood of being influenced by interfering substances, and unlikelihood of being influenced by solvents. Specifically, the present invention provides a modified protein prepared by obtaining various genes for various antibodies with environmental hormones, and modifying, by gene recombination technology, various properties of the original antibody, such as affinity, avidity, cross-reactivity, resistance for interfering substance on antigen-antibody reaction, resistance for interfering substance on enzymatic color developing reaction, resistance for solvent and the like.

## Description

### Technical Field

The present invention relates to an environmental hormone antibody, a gene encoding the same, a process of producing a protein capable of binding to said environmental hormones, a method of determining or quantifying environmental hormones, a method of concentrating environmental hormones and the like.

### Background Art

Environmental hormones are also referred to as "endocrine disruptors" or "endocrine disrupting chemicals", and recently a social problem has been caused by environmental pollution due to them. Then, it is necessary to determine and analyze environmental pollutants and their degradation products in an environment, and to utilize the results for environmental preservation.

As such measurement and analysis methods, the method described in WO99/43799, JP-A-2001-41958 is a superior method.

### Disclosure of Invention

The present invention aims at preparing and utilizing a protein having an avidity to environmental hormones, which is obtained by supplementing a modified protein prepared by obtaining genes for various antibodies against environmental hormones, and modifying, by gene recombination technology, various properties of the original antibody, such as affinity, avidity, cross-reactivity, resistance for interfering substance on antigen-antibody reaction, resistance for interfering substance on enzymatic color developing reaction, resistance for solvent and the like, with useful properties for determining, quantifying or concentrating environmental hormones, such as high sensitivity, low cross-reactivity, high tolerance for interfering substances, high tolerance for solvents and the like.

As used herein, environmental hormones include, for example, alkylphenol ethoxylates (APE) represented by the formula (1): 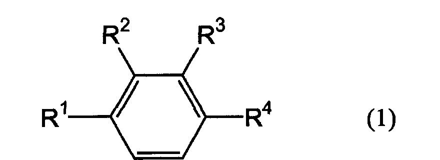 wherein R¹, R² and R³ are the same or different and each is H or a straight or branched (including sec-, tert-, iso-) alkyl group having 1 to 20, preferably 1 to 12 carbon atoms; R⁴ is (OC₂H₄)ₘOH or (OC₂H₄)ₘCOOH; and m is the average number of ethylene oxide chain of 1 to 70, preferably 1 to 15 [e.g., NPE (nonylphenol ethoxylate, such as NP1EO (ethylene oxide chain number=1), NP2EO (ethylene oxide chain number=2), NP5EO (the average number of ethylene oxide chain=5), NP6EO (nonylphenolhexaethoxylate: ethylene oxide chain=6), NP10EO (the average number of ethylene oxide chain=10), NP15EO (the average number of ethylene oxide chain=15), NP1EC (the average number of ethylene oxide chain=1), NP2EC (the average number of ethylene oxide chain=2), NP4EC (the average number of ethylene oxide chain=4), NP10EC (the average number of ethylene oxide chain=10, the terminal OH → carboxylic acid), OPE (octylphenol ethoxylate), and the like], alkylphenols (AP) represented by the formula (2): 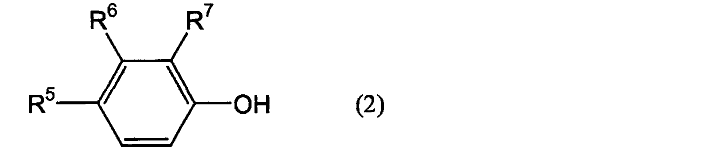 wherein R⁵, R⁶ and R⁷ are the same or different and each is H or a straight or branched (including sec-, tert-,iso-) alkyl group having 1 to 20, preferably 1 to 12 carbon atoms [e.g., DP (4-dodecylphenol), EP (4-ethylphenol), HP (heptylphenol), IPP (4-isopentylphenol), 2-OP (2-octylphenol), 4-NP (4-nonylphenol), 4-OP (4-octylphenol), 4-sBP (4-sec-butylphenol), 4-tBP (4-tert-butylphenol), 4-tPP (4-tert-pentylphenol), 4-tOP (4-tert-octylphenol), and the like],
Plastic resin components (PRC) represented by the formula (3): 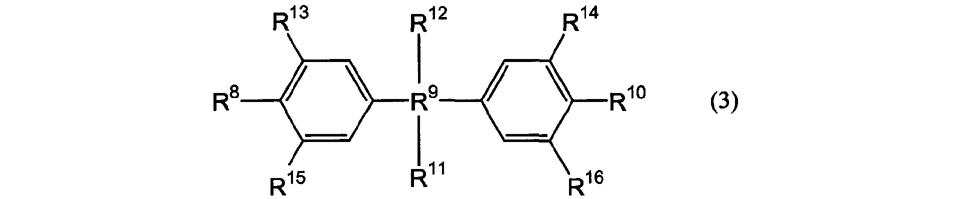 wherein R⁹ is C, CO or SO₂, R⁸ and R¹⁰ are the same or different and each is H, OH, NH₂ or O(CH₂) ₂OH, R¹¹ and OR¹² are the same or different and each is absent, H, CH₃, CH₂OH or C₂H₄COOH, R¹³, R¹⁴, R¹⁵ and R¹⁶ are the same or different and each is H, OH, CH₃, Cl or Br [e.g., BPA (bisphenol A), 4,4'-EBP (4,4'-ethylidenebisphenol), BHPM (bis(p-hydroxyphenyl)methane), 2,2'-BHPP (2,2'-bis(4-hydroxyphenyl)-1-propanol), 2,2'-BMHPP (2,2'-bis(m-methyl-p-hydroxyphenyl)propane), 4,4'-BHPP (4,4'-bis(p-hydroxyphenyl)pentanoic acid), 4,4'-DDM (4,4'-diaminodiphenylmethane), 4,4'-DDE (4,4'-dihydroxydiphenylether), 4,4'-DOHDS (4,4'-dihydroxydiphenylsulfone), 4,4'-DCIDS (4,4'-dichlorodiphenylsulfone), BHEDBrPS (bis[4-(2-hydroxyethoxy)-3,5-dibromophenyl]sulfone), BHEPS (bis[4-(2-hydroxyethoxy)phenyl]sulfone), 4,4'-DDE (4,4'-dihydroxydephenylether), p,p'-DBP (p,p'-dihydroxybenzophenone), HBP (4-hydroxybiphenyl), and the like],
Plastic resin plasticizers (PP) represented by the formula (4) : 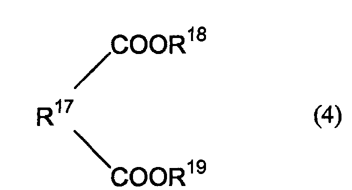 wherein R¹⁷ is o-phenylene or tetramethylene, R¹⁸ and R¹⁹ are the same or different and each is H, straight or branched (including sec-, tert-, iso-) alkyl having 1 to 20, preferably 1 to 12 carbon atoms, benzyl or cyclohexyl [e.g., BBP ((butyl benzyl phthalate), DBP (dibutyl phthalate), DCHP (dicyclohexyl phthalate), DEP (diethyl phthalate), DEHP (di(2-ethylhexyl) phthalate), DEHA (diethylhexyl adipate), DHP (dihexyl phthalate), DPP (di-n-pentyl phthalate), DPrP (dipropyl phthalate), and the like)), and
Chlorophenols (CP) represented by the formula (5): 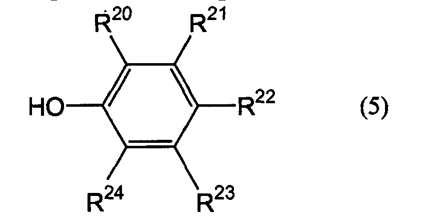 wherein R²⁰, R²¹, R²², R²³ and R²⁴ are the same or different and each is H or Cl [e.g., 2-CP (2-chlorophenol), 3-CP (3-chlorophenol), 4-CP (4-chlorophenol), 2,3-CP (2,3-dichlorophenol), 2,4-CP (2,4-dichlorophenol), 2,5-CP (2,5-dichlorophenol),2,6-CP (2,6-dichlorophenol), 2,3,4-CP (2,3,4-trichlorophenol), 2,4,5-CP (2,4,5-trichlorophenol), 2,4,6-CP (2,4,6-trichlorophenol), 2,3,4,5-CP (2,3,4,5-tetrachlorophenol), 2,3,4,6-CP (2,3,4,6-tetrachlorophenol), PCP (pentachlorophenol), and the like] can be mentioned. These compounds can be appropriately selected according to particular environmental hormones and a degradation products thereof to be analyzed or concentrated.

In addition, environmental contaminants (e.g., PCB, benzophenone, benzopyrane, chlorobenzene, bromonaphthol, nitrotoluene, dioxin, chlorobenzofuran, tributyltin etc.), various agrochemicals, heavy metals (e.g., Cd, Hg, Pb etc.), synthetic estrogens (e.g., centchroman, ethynylestradiol, diethylstilbestrol, hexestrol, 2-hydroxyestradiol, tamoxifen, raloxifene etc.)), foods, food additives (e.g., BHA (butylhydroxyanisole), equol, enterolactone, phytoestrogen, coumestrol, formononetin, daidzein, biotinin A, gestenin etc.) and the like are also encompassed in the environmental hormones in the present invention.

The present inventors have conducted intensive studies of obtaining a protein having avidity to environmental hormones, which is imparted with useful properties such as being capable of high sensitive measurement and the like, by improving affinity, and found that the protein having an avidity to environmental hormones can be efficiently produced by preparing a transformant containing its gene or an engineered gene, and further studied to complete the present invention.

Accordingly, the present invention provides
(1) a protein of the following [a] or [b], or a salt thereof:
   [a] a protein containing a heavy chain variable region of an anti-environmental hormone antibody, which has an amino acid sequence shown by SEQ ID No: 2, an amino acid sequence shown by SEQ ID No: 6, or an amino acid sequence substantially the same as these;
   [b] a protein containing a light chain variable region of an anti-environmental hormone antibody, which has an amino acid sequence shown by SEQ ID No: 4, an amino acid sequence shown by SEQ ID No: 8, or an amino acid sequence substantially the same as these;
(2) a protein of any of the following [a¹]-[a³] and [b¹]-[b³], or a salt thereof:
   [a¹] a protein consisting of an amino acid sequence of the following (i) or (ii), wherein a particular region is selected from the group consisting of a complementarity determining region 1, a complementarity determining region 2, a complementarity determining region 3, a framework region 1, a framework region 2, a framework region 3 and a framework region 4,
      (i) an amino acid sequence wherein an amino acid sequence corresponding to not less than one particular region of the amino acid sequence shown by SEQ ID No: 2 has been exchanged for an amino acid sequence corresponding to the same kind of not less than one particular region contained in an amino acid sequence shown by SEQ ID No: 6,
      (ii) an amino acid sequence wherein an amino acid sequence corresponding to not less than one particular region of the amino acid sequence shown by SEQ ID No: 6 has been exchanged for an amino acid sequence corresponding to the same kind of not less than one particular region contained in an amino acid sequence shown by SEQ ID No: 2,
   [b¹] a protein having an amino acid sequence of the following
      (i) or (ii), and a particular region selected from the group consisting of a complementarity determining region 1, a complementarity determining region 2, a complementarity determining region 3, a framework region 1, a framework region 2, a framework region 3 and a framework region 4,

      (i) an amino acid sequence wherein an amino acid sequence corresponding to not less than one particular region of the amino acid sequence shown by SEQ ID No: 4 has been exchanged for an amino acid sequence corresponding to the same kind of not less than one particular region contained in an amino acid sequence shown by SEQ ID No: 8,
      (ii) an amino acid sequence wherein an amino acid sequence corresponding to not less than one particular region of the amino acid sequence shown by SEQ ID No: 8 has been exchanged for an amino acid sequence corresponding to the same kind of not less than one particular region contained in an amino acid sequence shown by SEQ ID No: 4;
   [a²] a protein having an amino acid sequence shown by SEQ ID No: 2, an amino acid sequence shown by SEQ ID No: 6 or an amino acid sequence of the protein of [a¹], wherein one or more amino acids have been deleted, substituted or added, which binds to an environmental hormone upon formation of a complex with a protein having an amino acid sequence shown by SEQ ID No: 4, an amino acid sequence shown by SEQ ID No: 8 or an amino acid sequence of the protein of [b¹];
   [b²] a protein having an amino acid sequence shown by SEQ ID No: 4, an amino acid sequence shown by SEQ ID No: 8 or an amino acid sequence of the protein of [b¹], wherein one or more amino acids have been deleted, substituted or added, which binds to an environmental hormone upon formation of a complex with a protein having an amino acid sequence shown by SEQ ID No: 2, an amino acid sequence shown by SEQ ID No: 6 or an amino acid sequence of the protein of [a¹];
   [a³] a protein having an amino acid sequence shown by SEQ ID No: 2, an amino acid sequence shown by SEQ ID No: 6 or an amino acid sequence of the protein of [a¹], wherein one or more amino acids have been deleted, substituted or added, which binds to an environmental hormone upon formation of a complex with a protein having an amino acid sequence shown by SEQ ID No: 4, an amino acid sequence shown by SEQ ID No: 8 or an amino acid sequence of the protein of [b¹], wherein one or more amino acids have been deleted, substituted or added;
   [b³] a protein having an amino acid sequence shown by SEQ ID No: 4, an amino acid sequence shown by SEQ ID No: 8 or an amino acid sequence of the protein of [b¹], wherein one or more amino acids have been deleted, substituted or added, which binds to an environmental hormone upon formation of a complex with a protein having an amino acid sequence shown by SEQ ID No: 2, an amino acid sequence shown by SEQ ID No: 6 or an amino acid sequence of the protein of [a¹], wherein one or more amino acids have been deleted, substituted or added;
(3) the protein of the above-mentioned (1) or (2), or a salt thereof, wherein the environmental hormone is an alkylphenolethoxylate, an alkylphenol, a Plastic resin component, a plastic resin plasticizer or a chlorophenol;
(4) the protein of the above-mentioned (3), wherein the environmental hormone is an alkylphenol ethoxylate represented by the formula (1): 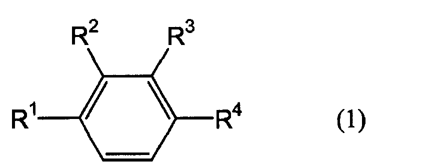 wherein R¹, R² and R³ are the same or different and each is H or a straight or branched alkyl group having 1 to 20 carbon atoms, R⁴ is (OC₂H₄)ₘOH or (OC₂H₄)ₘCOOH, and m shows an average number of ethylene oxide chain 1 to 70;
(5) the protein of the above-mentioned (3), wherein the environmental hormone is an alkylphenol represented by the formula (2): 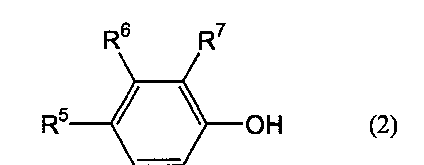 wherein R⁵, R⁶ and R⁷ are the same or different and each is H or a straight or branched alkyl group having 1 to 20 carbon atoms;
(6) a method of genetically engineering a protein of any of the above-mentioned (1)-(5);
(7) a protein obtained by the method of the above-mentioned (6),or a salt thereof;
(8) a partial peptide of a protein of any of the above-mentioned (1)-(5) and (7), or a salt thereof;
(9) a polynucleotide encoding a protein or partial peptide of a protein of any of the above-mentioned (1)-(5) and (7);
(10) a recombinant vector containing a polynucleotide of the above-mentioned (9);
(11) a transformant (e.g., transformants of deposit No.: FERM BP-7910 and deposit No.: FERM BP-7911) transformed with the recombinant vector of the above-mentioned (10);
(12) a method of producing a protein of any of the above-mentioned (1)-(5) and (7), or a partial peptide thereof, or a salt thereof, which comprises producing a protein of any of the above-mentioned (1)-(5) and (7), or a partial peptide thereof, or a salt thereof, and harvesting the same;
(13) a complex wherein a protein of the following [a] and a protein of the following [b] are linked:
   [a] a protein having an amino acid sequence shown by SEQ ID No: 2, an amino acid sequence shown by SEQ ID No: 6, or an amino acid sequence substantially the same as these;
   [b] a protein having an amino acid sequence shown by SEQ ID No: 4, an amino acid sequence shown by SEQ ID No: 8, or an amino acid sequence substantially the same as these;
(14) a method of identifying an environmental hormone binding to a complex of the above-mentioned (13), which comprises using said complex or a protein of any of the above-mentioned (1)-(5) and (7);
(15) a method of determining or quantifying an environmental hormone, which comprises using the complex of the above-mentioned (13) or a protein of any of the above-mentioned (1)-(5) and (7);
(16) a kit for determining or quantifying an environmental hormone, which comprises the complex of the above-mentioned (13) or a protein of any of the above-mentioned (1)-(5) and (7);
(17) a method of concentrating an environmental hormone, which comprises using the complex of the above-mentioned (13) or a protein of any of the above-mentioned (1)-(5) and (7);
(18) a kit for concentrating an environmental hormone, which comprises the complex of the above-mentioned (13) or a protein of any of the above-mentioned (1)-(5) and (7); and the like.

### Brief Description of Drawings

Fig. 1 is a conceptual drawing of 8 kinds of anti-environmental hormone single chain variable fragment antibodies constructed in the present invention.
Fig. 2 shows western blots of 8 kinds of anti-environmental hormone single chain variable fragment antibodies constructed in the present invention.

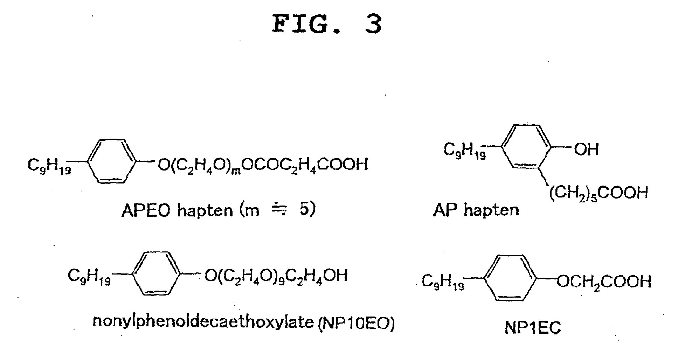

Fig. 3 shows the chemical structure of the representative compound used for indirect competition ELISA.
Fig. 4 shows comparison of the properties of 8 kinds of different single chain variable fragment antibodies prepared in the present invention by ELISA, wherein the abbreviations are same as described in Examples.

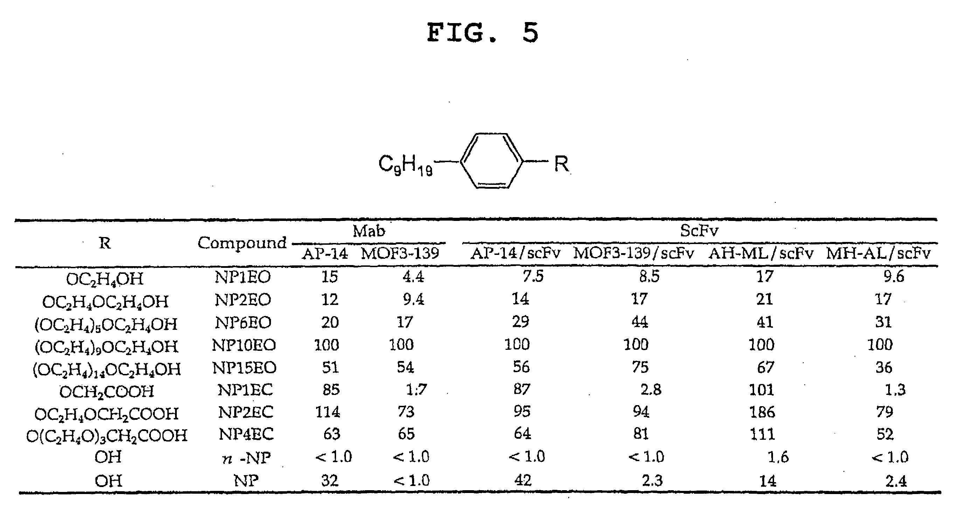

Fig. 5 shows cross-reactivity of monoclonal antibody and single chain variable fragment antibody to nonylphenol compounds, wherein the results are shown by cross-reactivity (%) = [IC₅₀ (µg/L) of NPnEO (n≒10) /IC₅₀ (µg/L) of test compound]× 100, Mab AP-14 shows an anti-alkylphenol monoclonal antibody and Mab MOF3-139 shows an anti-alkylphenol ethoxylate monoclonal antibody. In addition, AP-14scFv shows a single chain variable fragment antibody containing heavy chain variable region (VH) and light chain variable region (VL) of an anti-alkylphenol monoclonal antibody and MOF3-139scFv shows a single chain variable fragment antibody containing VH and VL of an anti-alkyl phenol ethoxylate monoclonal antibody. AH-MLscFv shows recombinant scFv containing VH of an anti-alkylphenol monoclonal antibody and VL of an anti-alkylphenol ethoxylate monoclonal antibody, MH-ALscFv shows recombinant scFv containing VH of an anti-alkylphenol ethoxylate monoclonal antibody and VL of an anti-alkylphenol monoclonal antibody. Moreover, n-NP shows normal nonylphenol and NP shows nonylphenol.

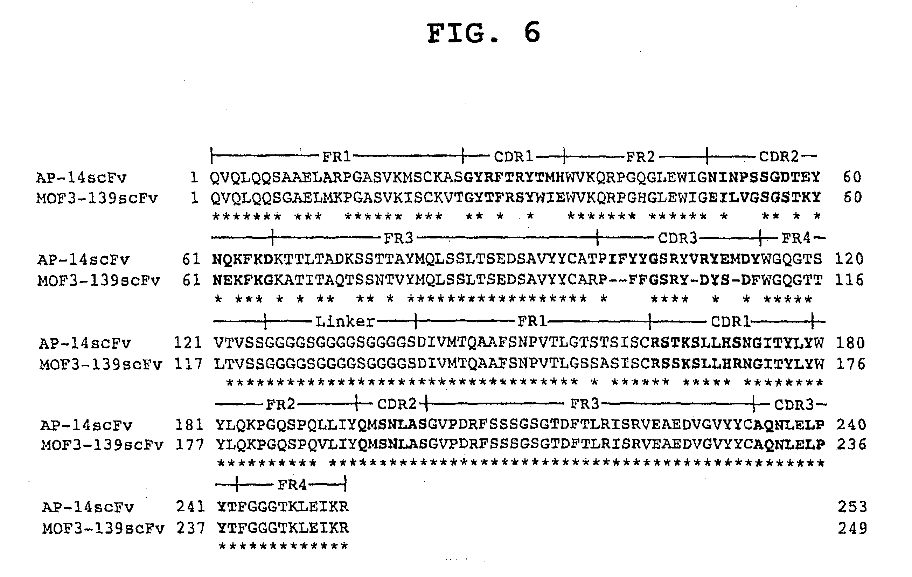

Fig. 6 shows alignment of amino acid sequences in the variable regions of AP-14scFv and MOF3-139scFv, wherein an asterisk means that the amino acids in both sequences are the same, gap is inserted to maximize the alignment and amino acids in CDR are indicated with by boldface. AP-14scFv shown in Fig. 6 contains, from N-terminal toward C-terminal, an amino acid sequence shown by SEQ ID No: 2, three repeats of an amino acid sequence shown by SEQ ID No: 24, and an amino acid sequence shown by SEQ ID No: 4, and MOF3-139scFv contains an amino acid sequence shown by SEQ ID No: 6, three repeats of an amino acid sequence shown by SEQ ID No: 24 and an amino acid sequence shown by SEQ ID No: 8.
Fig. 7 shows an outline of MOF3-139scFv mutant incorporating CDR or FR of AP-14scFv.

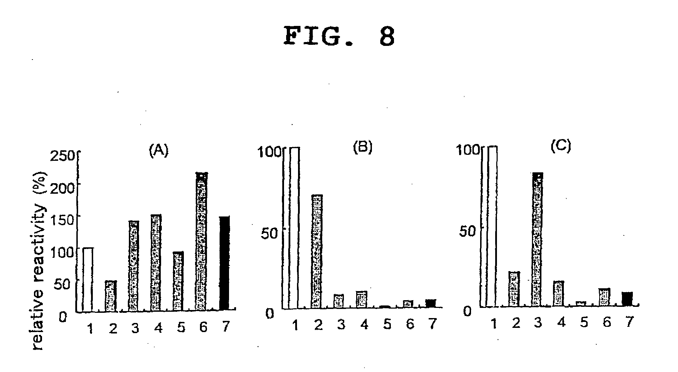

Fig. 8 shows relative reactivity of MOF3-139scFv mutant incorporating CDR or FR of AP-14scFv to NP10EO, NP1EC and NP, wherein the results are shown by relative reactivity (%)=[IC₅₀ of AP-14scFv/IC₅₀ of tested scFv]×100 and 1-7 are as shown below: 1, AP-14scFv; 2, MOF3-139scFv(CDR1) mutant; 3, MOF3-139scFv (CDR2) mutant; 4, MOF3-139scFv (CDR3) mutant; 5, MOF3-139scFv (FR1) mutant; 6, MOF3-139scFv (FR3) mutant; 7, MOF3-139scFv. The regions in the parentheses are the regions of AP-14scFv incorporated into MOF3-139scFv. A nonylphenoldecaethoxylate (NP10EO) standard solution was used in (A), a nonylphenoxyacetic acid (NP1EC) standard solution was used in (B), and a nonylphenol (NP) standard solution was used in (C).

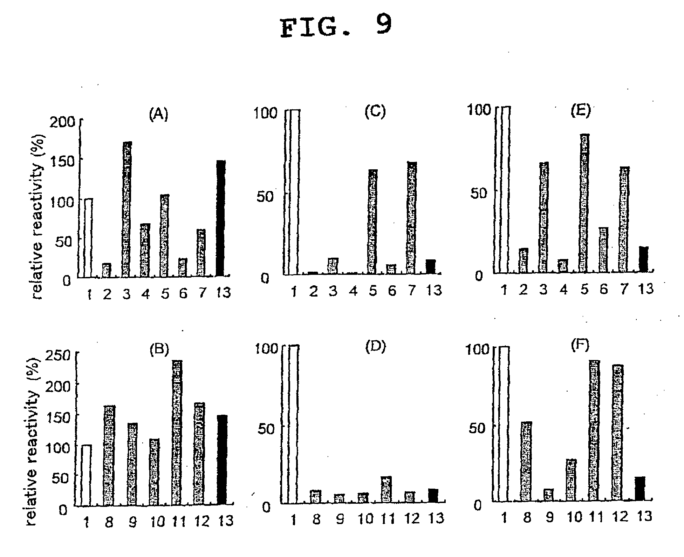

Fig. 9 shows relative reactivity of MOF3-139scFv mutant after point mutation to NP10EO, NP1EC and NP, wherein the results are shown by relative reactivity (%) = [IC₅₀ of AP-14scFv/IC₅₀ of tested scFv]×100 and 1-7 are as shown below: 1, AP-14scFv; 2, MOF3-139scFv(T28R) mutant; 3, MOF3-139scFv(R30T) mutant; 4, MOF3-139scFv(S31R) mutant; 5, MOF3-139scFv(W33T) mutant; 6, MOF3-139scFv(I34M) mutant; 7, MOF3-139scFv(E35H) mutant; 8, MOF3-139scFv(L52N) mutant; 9, MOF3-139scFv(V53P) mutant; 10, MOF3-139scFv(G54R) mutant; 11, MOF3-139scFv(S57D) mutant; 12, MOF3-139scFv(K59E) mutant; 13, MOF3-139scFv. The parentheses show amino acid residues changed in MOF3-139scFv. Moreover, a nonylphenoldecaethoxylate (NP10EO) standard solution was used in (A) and (B), a nonylphenoxyacetic acid (NP1EC) standard solution was used in (C) and (D) and a nonylphenol (NP) standard solution was used in (E) and (F).

### Detailed Description of the Invention

The present invention provides [a] a protein having (or consisting of) an amino acid sequence shown by SEQ ID No: 2, an amino acid sequence shown by SEQ ID No: 6, or an amino acid sequence substantially the same as these, or [b] a protein having (or consisting of) an amino acid sequence shown by SEQ ID No: 4, an amino acid sequence shown by SEQ ID No: 8, or an amino acid sequence substantially the same as these. The above-mentioned protein [a] is a protein having an amino acid sequence of a heavy chain variable region of an anti-environmental hormone antibody or an amino acid sequence substantially the same as said amino acid sequence. On the other hand, the above-mentioned protein [b] is a protein having an amino acid sequence of a light chain variable region of an anti-environmental hormone antibody or an amino acid sequence substantially the same as said amino acid sequence. These are sequentially explained in the following.

The above-mentioned protein [a] is not particularly limited as long as it has an amino acid sequence of a heavy chain variable region of an anti-environmental hormone antibody or an amino acid sequence substantially the same as said amino acid sequence. In one embodiment, the above-mentioned protein [a] can be a protein of the above-mentioned [a¹], [a²] or [a³]. In addition, the protein of the above-mentioned [a¹] is not particularly limited as long as it is a protein consisting of an amino acid sequence wherein particular regions of the amino acid sequence shown by SEQ ID No: 2 or the amino acid sequence shown by SEQ ID No: 6 are exchanged for particular regions of a different amino acid sequence, and a protein consisting of the above-mentioned (i) or (ii) can be specifically mentioned.

As the particular region in the above-mentioned [a¹], complementarity determining region 1, complementarity determining region 2, complementarity determining region 3 (hereinafter to be abbreviated as CDR1, CDR2, CDR3 as necessary), framework region 1, framework region 2, framework region 3, framework region 4 (hereinafter to be abbreviated as FR1, FR2, FR3, FR4 as necessary) can be mentioned. In the above-mentioned [a¹], the amino acid sequence to be the object of change is preferably an amino acid sequence of the same kinds of particular regions. Therefore, for example, a protein having an amino acid sequence corresponding to CDR1 in an amino acid sequence shown by SEQ ID No: 6, and an amino acid sequence corresponding to CDR2, CDR3, FR1, FR2, FR3, FR4 in an amino acid sequence shown by SEQ ID No: 2 is provided. In addition, while the number of the particular regions to be changed is not particularly limited as long as it is not less than 1, it is, for example, 1 to 3, preferably 1 or 2, more preferably 1. The amino acid sequences can be exchanged by a method known per se. Specifically, a primer wherein a part corresponding to the regions to be changed is linked to a primer corresponding to both the N, C terminals of each region is designed, and using this primer, a fragment is amplified by PCR, and a PCR is performed with the exchanged combination.

In SEQ ID No: 2 or an amino acid sequence shown by SEQ ID No: 6, the regions corresponding to CDR1, CDR2, CDR3, FR1, FR2, FR3, FR4 are specifically as follows:
(i) CDR1 (26th to 35th amino acid residues of an amino acid sequence shown by SEQ ID No: 2, and 26th to 35th amino acid residues of an amino acid sequence shown by SEQ ID No: 6);
(ii) CDR2 (50th to 66th amino acid residues of an amino acid sequence shown by SEQ ID No: 2, and 50th to 66th amino acid residues of an amino acid sequence shown by SEQ ID No: 6);
(iii) CDR3 (99th to 114th amino acid residues of an amino acid sequence shown by SEQ ID No: 2, and 99th to 110th amino acid residues of an amino acid sequence shown by SEQ ID No: 6);
(iv) FR1 (1st to 25th amino acid residues of an amino acid sequence shown by SEQ ID No: 2, and 1st to 25th amino acid residues of an amino acid sequence shown by SEQ ID No: 6);
(v) FR2 (36th to 49th amino acid residues of an amino acid sequence shown by SEQ ID No: 2, and 36th to 49th amino acid residues of an amino acid sequence shown by SEQ ID No: 6);
(vi) FR3 (67th to 98th amino acid residues of an amino acid sequence shown by SEQ ID No: 2, and 67th to 98th amino acid residues of an amino acid sequence shown by SEQ ID No: 6);
(vii) FR4 (115th to 125th amino acid residues of an amino acid sequence shown by SEQ ID No: 2, and 111th to 121st amino acid residues of an amino acid sequence shown by SEQ ID No: 6).

In another embodiment, the above-mentioned protein [a] is, for example, [a⁴] a protein having an amino acid sequence having significant homology to an amino acid sequence shown by SEQ ID No: 2, an amino acid sequence shown by SEQ ID No: 6 or an amino acid sequence of the protein of [a¹] above, which binds to an environmental hormone upon formation of a complex with a protein having an amino acid sequence having significant homology to an amino acid sequence shown by SEQ ID No: 4, an amino acid sequence shown by SEQ ID No: 8 or an amino acid sequence of the protein of [b] above.

The above-mentioned protein [b] is not particularly limited as long as it has an amino acid sequence of a light chain variable region of an anti-environmental hormone antibody or an amino acid sequence substantially the same as said amino acid sequence. In one embodiment, the above-mentioned protein [b] can be a protein of the above-mentioned [b¹], [b²] or [b³] . In addition, the protein of the above-mentioned [b¹] is not particularly limited as long as it is a protein consisting of an amino acid sequence wherein particular regions of the amino acid sequence shown by SEQ ID No: 4 or the amino acid sequence shown by SEQ ID No: 8 are exchanged for particular regions of a different amino acid sequence, and a protein consisting of the above-mentioned (i) or (ii) can be specifically mentioned.

As the particular regions in the above-mentioned [b¹], CDR1, CDR2, CDR3, FR1, FR2, FR3 and FR4 can be mentioned. In the above-mentioned [b¹], the amino acid sequence to be the object of change is preferably an amino acid sequence of the same kinds of particular regions. Therefore, for example, a protein having an amino acid sequence corresponding to CDR1 in an amino acid sequence shown by SEQ ID No: 4, and an amino acid sequence corresponding to CDR2, CDR3, FR1, FR2, FR3, FR4 in an amino acid sequence shown by SEQ ID No: 8 is provided. In addition, while the number of the particular regions to be changed is not particularly limited as long as it is not less than 1, it is, for example, 1 to 3, preferably 1 or 2, more preferably 1.

In an amino acid sequence shown by SEQ ID No: 4 or SEQ ID No: 8, the regions corresponding to CDR1, CDR2, CDR3, FR1, FR2, FR3, FR4 are specifically as follows:
(i) CDR1 (24th to 39th amino acid residues of an amino acid sequence shown by SEQ ID No: 4, and 24th to 39th amino acid residues of an amino acid sequence shown by SEQ ID No: 8);
(ii) CDR2 (55th to 61st amino acid residues of an amino acid sequence shown by SEQ ID No: 4, and 55th to 61st amino acid residues of an amino acid sequence shown by SEQ ID No: 8);
(iii) CDR3 (94th to 102nd amino acid residues of an amino acid sequence shown by SEQ ID No: 4, and 94th to 102nd amino acid residues of an amino acid sequence shown by SEQ ID No: 8);
(iv) FR1 (1st to 23rd amino acid residues of an amino acid sequence shown by SEQ ID No: 4, and 1st to 23rd amino acid residues of an amino acid sequence shown by SEQ ID No: 8);
(v) FR2 (40th to 54th amino acid residues of an amino acid sequence shown by SEQ ID No: 4, and 40th to 54th amino acid residues of an amino acid sequence shown by SEQ ID No: 8);
(vi) FR3 (62nd to 93rd amino acid residues of an amino acid sequence shown by SEQ ID No: 4, and 62nd to 93rd amino acid residues of an amino acid sequence shown by SEQ ID No: 8);
(vii) FR4 (103rd to 113th amino acid residues of an amino acid sequence shown by SEQ ID No: 4, and 103rd to 113th amino acid residues of an amino acid sequence shown by SEQ ID No: 8).

In another embodiment, the above-mentioned protein [b] is, for example, [b⁴] a protein having an amino acid sequence having significant homology to an amino acid sequence shown by SEQ ID No: 4, an amino acid sequence shown by SEQ ID No: 8 or an amino acid sequence of the protein of [b¹] above, which binds to an environmental hormone upon formation of a complex with a protein having an amino acid sequence having significant homology to an amino acid sequence shown by SEQ ID No: 2, an amino acid sequence shown by SEQ ID No: 6 or an amino acid sequence of the protein of [a] above.

In the present invention, the number of amino acids deleted, substituted or added in the amino acid sequence shown by any SEQ ID NO: X is not subject to limitation, as long as it is 1 or more than 1, and can for example, be 1 to 80, preferably about 1 to 20, more preferably about 1 to 9, still more preferably 1 to 5, and most preferably several (1 or 2).

In the present invention, amino acid substitution is not subject to limitation, as long as a particular amino acid is substituted with an optionally chosen amino acid, and can, for example, be conservative amino acid substitution or non-conservative amino acid substitution. "Conservative amino acid substitution" refers to substituting a particular amino acid with an amino acid having a side chain of similar nature. Specifically, in conservative amino acid substitution, a particular amino acid is substituted with another amino acid belonging to the same group. On the other hand, "non-conservative amino acid substitution" refers to substituting a particular amino acid with another amino acid having a side chain of different nature. Specifically, in non-conservative amino acid substitution, a particular amino acid is substituted with another amino acid belonging to a different group. Groups of amino acids having a side chain of similar nature are known in the art. As such groups of amino acids, for example, amino acids having a basic (i.e., positively charged) side chain (e.g., lysine, arginine, histidine), amino acids having an acidic (i.e., negatively charged) side chain (e.g., aspartic acid, glutamic acid), and amino acids having a neutral (i.e., uncharged) side chain (e.g., glycine, asparagine, glutamine, serine, threonine, tyrosine, cysteine, alanine, valine, leucine, isoleucine, proline, phenylalanine, methionine, tryptophan). Amino acids having a neutral side chain can further be classified into amino acids having a polar side chain (e.g., glycine, asparagine, glutamine, serine, threonine, tyrosine, cysteine) and amino acids having a non-polar side chain (e.g., alanine, valine, leucine, isoleucine, proline, phenylalanine, methionine, tryptophan). Other groups can include, for example, amino acids having an aromatic side chain (e.g., phenylalanine, tryptophan, histidine), amino acids having a side chain containing a hydroxyl group (alcoholic hydroxyl group, phenolic hydroxyl group) (e.g., serine, threonine, tyrosine), and the like.

As amino acid sequences having significant homology to the amino acid sequence shown by any SEQ ID NO: X, for example, amino acid sequences having homology to the amino acid sequence shown by any SEQ ID NO: X of about 40% or more, preferably about 60% or more, more preferably about 80% or more, still more preferably about 90% or more, and most preferably about 95% or more can be mentioned.

Degree of homology (%) can be determined by a method known *per se.* For example, degree of homology (%) can be determined using the Gap program employing the algorithm of Smith and Waterman (Adv. Appl. Math., 1981, 2, 482-489) (Wisconsin Sequence Analysis Package, Version 8 for Unix, Genetics Computer Group, University Research Park, Madison WI) in default settings. The BLAST program employing the algorithm of Karlin and Altschul (Proc. Natl. Acad. Sci. USA, 1990, 87:2264-2268, Proc. Natl. Acad. Sci. USA, 1993, 90:5873-5877) can also be used. For example, when comparing protein homology, degree of homology (%) can be determined using the XBLAST program in default settings. Furthermore, the ALIGN program (version 2.0) (a portion of GCG sequence alignment software package) employing the algorithm of Myers and Miller (CABIOS, 1988, 4:11-17) can also be used. As settings for comparing amino acid sequences using the ALIGN program, for example, "PAM120 weight residue table, gap length penalty = 12, gap penalty = 4" can be mentioned. These programs can also be used for determining degree of homology (%) of base sequences.

"Binding to an environmental hormone upon formation of a complex" means that the complex is reactive to the environmental hormone. As the environmental hormones, for example, the aforementioned alkylphenolethoxylates, alkylphenols, plastic resin components, plastic resin plasticizers and chlorophenols can be mentioned. Of these, the above-mentioned alkylphenolethoxylates and alkylphenols are preferable. Whether or not the complex is capable of binding to an environmental hormone can be determined by a method known *per se* such as the measuring method described below or a method based thereon. The complex of the present invention may be any one, as long as it is capable of binding to any of the aforementioned environmental hormones.

In an embodiment of the present invention, the proteins [a²]-[a⁴] or [b²]-[b⁴] above, which have varied binding capability or cross-reactivity for a environmental hormone, can be obtained by introducing a deletion, substitution or addition of 1 or more amino acids to a protein having the amino acid sequence shown by SEQ ID NO:2, the amino acid sequence shown by SEQ ID NO:4, the amino acid sequence shown by SEQ ID NO:6, the amino acid sequence shown by SEQ ID NO:8, or to the protein [a¹] or [b¹]. In the proteins [a²]-[a⁴] or [b²]-[b⁴] above, the region to accept a deletion, substitution or addition of 1 or more amino acids can be 1 or more regions selected from the group consisting of CDR1, CDR2, CDR3, FR1, FR2, FR3 and FR4.

For example, in the proteins of the above-mentioned [a²]-[a⁴] (particularly, a protein based on an amino acid sequence shown by SEQ ID No: 6), not less than one amino acids in one or more regions selected from the group consisting of CDR1, CDR2 and FR3 can be substituted by any other amino acids. Such substitution of the amino acids makes it possible to enhance avidity to particular environmental hormones, and change cross-reactivity with various environmental hormones.

To be specific, as substitution of amino acids in CDR1 of a protein based on an amino acid sequence shown by SEQ ID No: 6, the substitutions described in the following (1)-(6) can be mentioned. In addition, as substitution of amino acids in CDR2 of a protein based on an amino acid sequence shown by SEQ ID No: 6, the substitutions described in the following (7)-(11) can be mentioned. Moreover, as substitution of amino acids in FR3 of a protein based on an amino acid sequence shown by SEQ ID No: 6, the substitutions described in the following (12)-(13) can be mentioned. The substitution of amino acids described in the following (1)-(13) can be combined as appropriate. While amino acids other than the following (1)-(13) can be substituted by random amino acids, it may be, for example, preservative amino acid substitution.
(1) non-conservative amino acid substitution at 28th threonine in an amino acid sequence shown by SEQ ID No: 6, preferably, substitution by an amino acid having a basic side chain (e.g., arginine);
(2) non-conservative amino acid substitution at 30th arginine in an amino acid sequence shown by SEQ ID No: 6, preferably, substitution by an amino acid having a neutral side chain or a side chain containing hydroxyl group (e.g., threonine);
(3) non-conservative amino acid substitution at 31st serine in an amino acid sequence shown by SEQ ID No: 6, preferably, substitution by an amino acid having a basic side chain (e.g., arginine);
(4) non-conservative amino acid substitution at 33rd tryptophan in an amino acid sequence shown by SEQ ID No: 6, preferably, substitution by an amino acid having a polar side chain or a side chain containing hydroxyl group (e.g., threonine);
(5) substitution of methionine for 34th isoleucine in an amino acid sequence shown by SEQ ID No: 6;
(6) non-conservative amino acid substitution at 35th glutamic in an amino acid sequence shown by SEQ ID No: 6, preferably, substitution by an amino acid having a basic side chain or aromatic side chain (e.g., histidine);
(7) non-conservative amino acid substitution at 52nd leucine in an amino acid sequence shown by SEQ ID No: 6, preferably, substitution by an amino acid having a polar side chain (e.g., asparagine);
(8) substitution of proline for 53rd valine in an amino acid sequence shown by SEQ ID No: 6;
(9) non-conservative amino acid substitution at 54th glycine in an amino acid sequence shown by SEQ ID No: 6, preferably, substitution by an amino acid having a side chain containing a hydroxyl group (e.g., serine);
(10) non-conservative amino acid substitution at 57th serine in an amino acid sequence shown by SEQ ID No: 6, preferably, substitution by an amino acid having an acidic side chain (e.g., aspartic acid);
(11) non-conservative amino acid substitution at 59th lysine in an amino acid sequence shown by SEQ ID No: 6, preferably, substitution by an amino acid having an acidic side chain (e.g., glutamic acid);
(12) substitution of serine for 116th threonine in an amino acid sequence shown by SEQ ID No: 6;
(13) substitution of valine for 117th leucine in an amino acid sequence shown by SEQ ID No: 6;

In one embodiment, a substitution can be appropriately selected from those described in the above-mentioned (1)-(13) to improve affinity to a particular compound. When affinity to NP10EO is to be improved, for example, the substitution of the above-mentioned (2) or (10) and the like is preferable. When affinity to NP1EC is to be improved, the substitution described in the above-mentioned (4), (6) and the like is preferable. When affinity to NP is to be improved, the substitution described in the above-mentioned (2), (4), (6), (7), (10), (11) and the like is preferable. When affinity to NP is to be improved while suppressing affinity to NP1EC, the substitution described in the above-mentioned (7), (10), (11) and the like is more preferable.

The partial peptide of the present invention may be any, as long as it is a peptide that constitutes a portion of the protein [a] or [b] above; there may be used, for example, a peptide consisting of at least 6 or more, preferably at least 8 or more, more preferably at least 10 or more, still more preferably at least 12 or more, and most preferably 15 or more consecutive amino acids in the amino acid sequence of protein [a] or [b] above. As the partial peptide of the present invention, there can also be used a partial peptide having (or consisting of) an amino acid sequence corresponding to the CDR1, CDR2, CDR3, FR1, FR2, FR3, or FR4 of protein [a] or [b] above.

As salts of the protein of the present invention or partial peptide thereof, there may be used known salts, for example, acid adduct salts. These acid adduct salts include, for example, salts with inorganic acids (e.g., hydrochloric acid, phosphoric acid, hydrobromic acid, sulfuric acid) or salts with organic acids (e.g., acetic acid, formic acid, propionic acid, fumaric acid, maleic acid, succinic acid, tartaric acid, citric acid, malic acid, oxalic acid, benzoic acid, methanesulfonic acid and benzenesulfonic acid).

In the present invention, the "complex" may be any one, as long as protein [a] above and protein [b] above are linked together, and is exemplified by a complex wherein protein [a] above and protein [b] above are covalently bound in the presence or absence of a linker. This complex can also be used in the form of a salt (preferably an acid adduct salt) as with the aforementioned protein and partial peptide.

As the linker for fusing protein [a] above and protein [b] above, there may be used without limitation any linker known in the art; such linkers include, for example, peptides of repeated sequences of GGGS (SEQ ID NO:24), GSTSGSGKSSEGKG (SEQ ID NO:25), GSTSGSGKSSEGSGSTKG (SEQ ID NO:26), GSTSGKPSEGKG (SEQ ID NO:27), GSTSGSGKPGSGEGSTKG (SEQ ID NO:28), etc. [see, for example, Production of single-chain Fv monomers and multimers, D. Filpula, J. McGuire, and M. Whitlow. In "Antibody Engineering" edited by J. McCafferty, H. R. Hoogenboon, and D. J. Chiswell. pp. 253-268, IRL PRESS (1996)]. A complex wherein protein [a] above and protein [b] above are covalently bound in the presence or absence of a linker can, for example, be obtained by separately preparing protein [a] above and protein [b] above, and subsequently covalently binding these proteins via a linker or by a direct covalent linkage. However, this method is painstaking because it requires a step to link protein [a] above and protein [b] above after their preparation, so as to obtain their complex. Another drawback resides in that a plurality of complexes with different covalent bond sites can result in, so that a single complex that is preferred from the viewpoint of reproducibility etc. is difficult to prepare. Therefore, the complex of the present invention is, for example, preferably a single-chain antibody (herein also referred to as recombinant antibody, single chain variable fragment antibody etc.) wherein protein [a] above and protein [b] above are fused by an amide linkage via a peptide linker or by a direct amide linkage. Such a single-chain antibody is useful in that it can easily be prepared from a transformant carrying an expression vector that comprises the base sequence encoding protein [a] above, the base sequence encoding a peptide linker (when obtaining a single-chain antibody amide-bound via a linker), and the base sequence encoding protein [b] above in frame. The base sequence encoding a peptide linker may be any one, as long as it does not contain stop codon in frame with the base sequences encoding proteins [a] and [b] above.

A peptide linker can be selected as appropriate by a method known in the art. Specifically, as a peptide linker, there may be used peptides of optionally chosen length consisting of 1 or more amino acid residues; there may be used, for example, peptides consisting of 10 or more amino acid residues.

The present invention also provides a polynucleotide (herein understood to be the same as gene and DNA) encoding the amino acid sequence of the protein of the present invention. The polynucleotide of the present invention may be any one, as long as it contains the aforementioned base sequence encoding the protein of the present invention.

Specifically, as the polynucleotide of the present invention, there may be mentioned base sequences encoding protein [a] above (e.g., base sequence shown by SEQ ID NO:1 or SEQ ID NO:5), base sequences encoding protein [b] above (e.g., base sequence shown by SEQ ID NO:3 or SEQ ID NO:7), and base sequences encoding the single-chain antibody above. As the polynucleotide of the present invention, there may also be mentioned a polynucleotide having the base sequence encoding a protein obtained by gene recombination of the protein of the present invention.

The aforementioned gene (DNA, polynucleotide) of the present invention can be obtained using a method known per se according to the disclosure herein. For example, the gene of the present invention can be obtained from a hybridoma that produces an anti-environmental hormone monoclonal antibody, which is not to be construed as limitative. The N-terminal amino acid sequence of the antibody protein is determined, a primer having a base sequence deduced from this amino acid sequence is then prepared, mRNA is prepared from an antibody-producing hybridoma by a methods known *per se,* and single-stranded cDNA is synthesized on the basis of the mRNA using reverse transcriptase, after which the gene of the present invention can be obtained selectively using PCR, hybridization, etc. on the basis of the amino acid sequence or base sequence of the variable region of a heavy chain or light chain of an anti-environmental hormone monoclonal antibody disclosed herein. Such methods are well-known; those skilled in the art can easily isolate the gene of the present invention on the basis of the disclosure herein. As specific procedures for these methods, there may be mentioned, for example, the method described in Molecular Cloning, 3rd edition (J. Sambrook et. al., Cold Spring Harbor Lab. Press, 2001). Useful methods of mRNA extraction include the method described in the operating manual for the Amersham QuickPrep mRNA purification kit; useful methods of cDNA synthesis and 5'-RACE include the methods described in the instruction manual for the CLONTECH Laboratories SMART RACE kit.

Regarding how to prepare recombinant antibodies, Chapter 2 of "RECOMBINANT ANTIBODIES" [edited by F. Breitling, John Wiley & Sons (USA), 1999] describes a method of preparing recombinant antibody fragments, a method of cloning antibody genes from hybridoma cell lines, a method of preparing antibody gene libraries, a method of selection of recombinant antibodies from gene libraries, a method of antibody engineering, etc., using which methods recombinant antibodies can be prepared.

Chapter 4 of the same book describes a method of producing recombinant antibodies and recombinant antibody fragments, and methods of their expression, including expression in rabbit reticulocyte lysate in vitro; expression in prokaryotes such as E. coli cytoplasm, soluble fraction of periplasm, inclusion body of periplasm, Bacillus and Streptomyces; and also described are methods of expression in eukaryotes such as Pichia, Saccharomyces, Schizosaccharomyces and other yeasts, Trichoderma and other fungi; expression in insect cells such as Baculovirus; expression in animal cells such as myeloma, CHO, and COS, transgenic plants such as of tobacco, and transgenic animals, using which methods transformants can be prepared.

Chapter 4 of the same book also describes methods of purifying recombinant antibodies and recombinant antibody fragments, wherein the desired product is first separated by a physical means, for example, cell harvest by centrifuging transformant, cell disruption by ultrasonication etc., mechanical milling, or enzymatic lysis. Subsequently, purification is conducted using a combination of ion exchange chromatography, size exclusion chromatography, thiophilic adsorption chromatography, affinity chromatography, etc. Affinity chromatography, in particular, is an efficient method; the desired product can be produced by purification using an antigen-specific method based on antigen recognition specificity, an antibody-specific method based on binding to the Fc or Fab' portion of protein A or protein G, or in the case of scFv without these portions, a method comprising expressing the ScFv as a fusion antibody having a small peptide fragment called "tag" and an affinity column specific for this tag is used (e.g., His-tag, c-myc tag, Strep tag, etc.).

First, a cDNA library of anti-environmental hormone monoclonal antibody-producing cells is constructed, and the cDNA library is then screened using a cDNA encoding the N-terminal sequence of the highly conservative constant region or the variable region of a heavy or light chain of an immunoglobulin as a probe; anti-environmental hormone monoclonal antibody light or heavy chain cDNA can be thus isolated.

As specific procedures for these methods, there may be mentioned, for example, the method described in Molecular Cloning, 3rd edition (J. Sambrook et. al., Cold Spring Harbor Lab. Press, 2001).

The gene of the present invention may also be synthesized chemically using a well-known technique on the basis of the sequence described herein.

Methods of gene engineering of the protein of the present invention include methods known per se; there may be used, for example, methods of converting the base sequence encoding the protein. Conversion of DNA base sequences can be achieved by a method known *per se* such as the ODA-LAPCR method, the Gupped duplex method, or the Kunkel method, or a method based thereon, using PCR or a known kit such as Mutan™-Super Express Km (Takara Shuzo Co., Ltd.) or Mutan™-K (Takara Shuzo Co., Ltd.) and the like. Depending on the purpose of use, the cloned antibody protein-encoding DNA can be used as is, or after being digested with a restriction enzyme or added with a linker as necessary. This DNA may have ATG as a translation initiation codon on the 5'-terminus thereof and TAA, TGA or TAG as a translation stop codon on the 3'-terminus thereof. These translation initiation codons and translation stop codons can also be added using an appropriate synthetic DNA adapter. An expression vector for the antibody protein of the present invention can, for example, be produced by (a) cleaving the desired DNA fragment from the DNA encoding the antibody protein of the present invention, and (b) joining the DNA fragment to downstream of a promoter in an appropriate expression vector.

### Methods of preparing recombinant antibodies

Various forms of recombinant antibodies can be prepared and are exemplified by those described in Roland Kontermann's ANTIBODY ENGINEERING HOME PAGE (http://aximt1.imt.uni-marburg.de/~rek/AEP.html, February 25, 2002); for example, recombinant antibodies can be prepared in the form of Fab' fragments, F(ab') fragments, Fv fragments (Fv), single-chain Fv fragments (scFv), bispecific-chimeric scFV (χ-scFv), tandem scFV (scFv)2, bispecific-(scFv)2, disulfide-linked scFv, disulfide-stabilized Fv fragments (dsFv), diabody, single-chain diabody (scDb), bivalent diabody, bispecific diabody, knob-into-hole stabilized diabody, disulfide-stabilized diabody, triabody, tetrabody, trispecific triabody, CL-dimerized scFv, CH1-CL-dimerized scFv, CH3-dimerized scFv, knob-into-hole CH3-dimerized scFv, CH3-dimerized bivalent diabody, Fc-dimerized scFv, Fab-scFv fusions, Ig-scFv fusions, leucine-zipper stabilized scFv dimers, helix-stabilized scFv dimers, 4 helix-bunde stabilized scFv tetramers, streptavidin-scFv, and intrabody.

Methods wherein an antibody having a desired useful property is selected by shuffling mutated antibody genes also fall in the scope of the present invention.

### Recombinant antibody expression systems

Any recombinant antibody expression system can be used, as long as it is capable of efficiently expressing a recombinant antibody; for example, as tabulated in Roland Kontermann's ANTIBODY ENGINEERING HOME PAGE (http://aximt1.imt.uni-marburg.de/-rek/AEP.html, February 25, 2002), expression of Fv, scFv and scFv derivatives, bivalent and bispecific scFv, scFv or Fab-fusion proteins, intrabodies, etc. in mammalian cells, expression of scFV, Fab, etc. in insect cells, expression of Fv, scFv, Fab, etc. in fungal cells, and expression of scFv in plant cells are known, and hence a variety of expression systems can be used.

### Methods of constructing a cDNA library

Any method of constructing a cDNA library can be used, as long as it is capable of efficiently preparing a cDNA library; such methods include the phage display method described in Roland Kontermann's ANTIBODY ENGINEERING HOME PAGE (http://aximt1.imt.uni-marburg.de/∼rek/AEP.html, February 25, 2002).

### Selection methods of recombinant antibody

As useful methods of selecting the desired recombinant antibody from the library constructed, the methods described in Roland Kontermann's ANTIBODY ENGINEERING HOME PAGE (http://aximt1.imt.uni-marburg.de/-rek/AEP.html, February 25, 2002), such as the protocol "Isolation of Recombinant Antibodies from Phagemid Libraries" and "Isolation of Peptides from fd Phage Libraries" can be mentioned.

The gene (DNA) used for expression may have substantially the same amino acid sequence as the anti-environmental hormone antibody protein expressed by the gene (DNA) obtained as described above, or a partial peptide thereof. Substantially the same amino acid sequence is exemplified by amino acid sequences with a partial modification (substitution), with an amino acid sequence added, or with an amino acid sequence removed (deleted).

Depending on the purpose of use, the gene (DNA) can be used as is, or after being cleaved or added with another DNA as necessary. This DNA may have a translation initiation codon ATG on the terminal thereof. This can be achieved by a methods known *per se.* Such methods include, for example, the method described in Molecular Cloning, 3rd edition (J. Sambrook et. al., Cold Spring Harbor Lab. Press, 2001).

By incorporating the thus-obtained DNA, a promoter, a translation initiation codon, the appropriate signal sequence, etc., by a methods known *per se*, into a vector, a recombinant vector can be produced.

As such vectors, promoters and host strains, there may be mentioned, for example, the vectors, promoters and Escherichia strains described in Appendix 3 to Molecular Cloning, 3rd edition (J. Sambrook et. al., Cold Spring Harbor Lab. Press, 2001).

In addition to the aforementioned vectors, useful vectors include plasmids of *Escherichia coli* origin (pET-276, pCANTAB-5E, pUC19, pT7Bule T), plasmids of *Bacillus subtilis* origin (e.g., pUB110, pTP5, pC194), plasmids of yeast origin (e.g., pSH19, pSH15), λ phage, bacteriophages such as M13K07, and animal viruses such as retrovirus, vacciniavirus and vaculovirus, as well as pA1-11, pXT1, pRc/CMV, pRc/RSV, pcDNAI/Neo and the like.

Any promoter can be used, as long as it is suitable for the host used for gene expression. Preferred promoters include, for example, the trp promoter, the lac promoter, the recA promoter, the λP_{L} promoter, and the lpp promoter when the host is a bacterium of the genus *Escherichia*; the SPO1 promoter, the SPO2 promoter, and the penP promoter when the host is a bacterium of the genus *Bacillus;* and the PHO5 promoter, the PGK promoter, the GAP promoter, and the ADH promoter when the host is a yeast. When the host is an animal cell, the SRα promoter, the SV40 promoter, the LTR promoter, the CMV promoter, the HSV-TK promoter, etc. are preferred; when the host is an insect cell, the polyhedrin promoter, the P10 promoter, etc. are preferred.

In addition to the aforementioned promoters, the expression vector may contain an enhancer, splicing signal, polyA addition signal, selection marker, SV40 replication origin, etc. as necessary. Selection markers include, for example, the ampicillin resistance gene (hereinafter also referred to as Amp^{R}), the kanamycin resistance gene (hereinafter also referred to as Km^{R}), and the chloramphenicol resistance gene (hereinafter also referred to as Cm^{R}).

Where necessary, a signal sequence suitable for the host is added to the N-terminus side of the antibody protein of the present invention. Useful signal sequences include the phoA signal sequence, the ompA signal sequence and the like when the host is a bacterium of the genus *Escherichia;* the α-amylase signal sequence, the subtilisin signal sequence and the like when the host is a bacterium of the genus *Bacillus;* the MFα signal sequence, the SUC2 signal sequence and the like when the host is a yeast; and the insulin signal sequence, the α-interferon signal sequence, and the antibody molecule signal sequence and the like when the host is an animal cell. Using the thus-constructed vector harboring the DNA encoding the antibody protein of the present invention, a transformant can be produced.

Useful hosts include the genus *Escherichia,* the genus *Bacillus,* yeasts, insect cells, insects, animal cells and the like. Examples of useful bacteria of the genus *Escherichia* include *Escherichia coli* K12-DH1 [Proc. Natl. Acad. Sci. USA, vol. 60, 160 (1968)], JM103 [Nucleic Acids Research, vol. 9, 309 (1981)], JA221 [Journal of Molecular Biology, vol. 120, 517 (1978)], HB101 [Journal of Molecular Biology, vol. 41, 459 (1969)], C600 [Genetics, vol. 39, 440 (1954)], BL21DE3(pLysS), TG-1, and JM109. Useful bacteria of the genus *Bacillus* include, for example, *Bacillus subtilis* MI114 [Gene, vol. 24, 255 (1983)] and 207-21 [Journal of Biochemistry, vol. 95, 87 (1984)]. Useful yeasts include, for example, *Saccharomyces cerevisiae* AH22, AH22R⁻, NA87-11A, DKD-5D, and 20B-12, *Schizosaccharomyces pombe* NCYC1913 and NCYC2036, and *Pichia pastoris.* When the virus is AcNPV, useful insect cells include, for example, established *Spodoptera frugiperda* larva cells (Sf cells), *Trichoplusia ni* midgut MG1 cells, *Trichoplusia ni* egg High Five™ cells, *Mamestrabrassicae* cells, *Estigmena acrea* cells and the like. When the virus is BmNPV, silkworm-derived established cells (*Bombyx mori* N; BmN cells) etc. are used. Such Sf cells include, for example, Sf9 cells (ATCC CRL1711) and Sf21 cells [both described by Vaughn, J.L. et al. In Vivo, 13, 213-217 (1977)]. Useful insects include, for example, silkworm larvae [Maeda et al., Nature, vol. 315, 592 (1985)]. Useful animal cells include, for example, simian COS-7 cells, Vero, Chinese hamster CHO cells, mouse L cells, mouse AtT-20, mouse myeloma cells, rat GH3 and human FL cells.

Transformation of bacteria of the genus Escherichia can, for example, be achieved according to the methods described in Proc. Natl. Acad. Sci. USA, vol. 69, 2110 (1972) and Gene, vol. 17, 107 (1982). Transformation of bacteria of the genus *Bacillus* can, for example, be achieved according to the method described in Molecular & General Genetics, vol. 168, 111 (1979). Transformation of yeasts can, for example, be achieved according to the methods described in Methods in Enzymology, vol. 194, 182-187 (1991) and Proc. Natl. Acad. Sci. USA, vol. 75, 1929 (1978). Transformation of insect cells or insects can, for example, be achieved according to the method described in Bio/Technology, vol. 6, 47-55 (1988). Transformation of an animal cell can, for example, be achieved using the method described in SAIBO KOGAKU (CELL TECHNOLOGY), Supplementary 8: *Shin Saibou Kougaku Jikken Protocol,* 263-267 (1995) (published by Shujunsha Co., Ltd.) and Virology, Vol. 52, 456 (1973).

Thus, a transformant transformed with an expression vector harboring the DNA encoding an antibody protein is obtained.

Furthermore, the protein of the present invention can be produced by cultivating the thus-obtained transformant and harvesting the resulting protein.

Regarding culture media, liquid media are suitable for the cultivation of a transformant whose host is a bacterium of the genus *Escherichia* or *Bacillus,* which media are supplemented with carbon sources, nitrogen sources, minerals, and other substances required for the growth of the transformant. Carbon sources include, for example, glucose, dextrin, soluble starch, sucrose and the like; nitrogen sources include, for example, inorganic or organic substances such as ammonium salts, nitrates, corn steep liquor, peptone, casein, meat extract, soybean flour, potato extract and the like; minerals include, for example, calcium chloride, sodium dihydrogen phosphate, magnesium chloride and the like. Yeast, vitamins, growth promoters, etc. may also be added. It is desirable that the pH of the medium be about 5 to 8.

Preferred media for cultivating the genus *Escherichia* include, for example, M9 medium supplemented with glucose and casamino acid [Miller, Journal of Experiments in Molecular Genetics, 431-433, Cold Spring Harbor Laboratory, New York (1972)]. To increase promoter efficiency where necessary, an agent like 3β-indolylacrylic acid or isopropylthiogalactoside (IPTG), for example, may be added. When the host is a bacterium of the genus *Escherichia,* cultivation is normally carried out at about 15 to 43°C for about 3 to 24 hours, and aeration and stirring may be conducted as necessary. When the host is a bacterium of the genus *Bacillus,* cultivation is normally carried out at about 30 to 40°C for about 6 to 24 hours, and aeration and stirring may be conducted as necessary. Media for cultivating a transformant whose host is a yeast include, for example, Burkholder's minimum medium [Bostian, K.L. et al., Proc. Natl. Acad. Sci. USA, vol. 77, 4505 (1980)] and SD medium supplemented with 0.5% casamino acid [Bitter, G.A. et al., Proc. Natl. Acad. Sci. USA, vol. 81, 5330 (1984)]. It is preferable that the medium's pH be adjusted to about 5 to 8. Cultivation is normally carried out at about 20 to 35°C for about 24 to 72 hours, with aeration and stirring conducted as necessary.

Useful media for cultivating a transformant whose host is an insect cell or an insect include Grace's insect medium [Grace, T.C.C., Nature, 195, 788 (1962)] supplemented with additives such as inactivated 10% bovine serum as appropriate. It is preferable that the medium's pH be adjusted to about 6.2 to 6.4. Cultivation is normally carried out at about 27°C for about 3 to 5 days, with aeration and stirring conducted as necessary. Useful media for cultivating a transformant whose host is an animal cell include, for example, MEM medium supplemented with about 5 to 20% fetal bovine serum [Science, vol. 122, 501 (1952)], DMEM medium [Virology, vol. 8, 396 (1959)], RPMI1640 medium [The Journal of the American Medical Association], vol. 199, 519 (1967)], and 199 medium [Proceeding of the Society for the Biological Medicine, vol. 73, 1 (1950)]. It is preferable that pH be about 6 to 8. Cultivation is normally carried out at about 30 to 40°C for about 15 to 60 hours, with aeration and stirring conducted as necessary. As described above, the antibody protein of the present invention can be produced in the cells or cell membrane of the transformant or outside the cells.

Separation and purification of the desired antibody protein of the present invention from the thus-obtained culture can, for example, be achieved by the method described below. When extracting the antibody protein of the present invention from cultured cells, there may be used as appropriate, for example, a method wherein cultured cells are collected by a known means, suspended in an appropriate buffer solution, and disrupted by means of ultrasound, lysozyme and/or freeze-thawing etc., after which a crude extract of antibody protein is obtained by centrifugation or filtration. The buffer solution may contain a protein denaturant such as urea or guanidine hydrochloride and a surfactant such as Triton X-100™. If the antibody protein is secreted in the culture broth, it is treated by a method known per se to separate the cells and the supernatant after completion of cultivation and the supernatant is collected. Purification of the antibody protein contained in the thus-obtained culture supernatant or extract can be achieved by appropriately combining methods known *per se* of separation and purification. Useful known methods of separation and purification include methods based on solubility, such as salting-out and solvent precipitation; methods based mainly on molecular weight differences, such as dialysis, ultrafiltration, gel filtration, and SDS-polyacrylamide gel electrophoresis; methods based on charge differences, such as ion exchange chromatography; methods based on specific affinity, such as affinity chromatography; methods based on hydrophobicity differences, such as reversed-phase high performance liquid chromatography; and methods based on isoelectric point differences, such as isoelectric focusing.

The protein of the present invention or a salt thereof can be produced by a method known per se of protein synthesis, or by cleaving the protein of the present invention using an appropriate protease. This protein synthesis can, for example, be achieved by solid phase synthesis or liquid phase synthesis. Specifically, the desired protein can be produced by condensing the partial peptide or amino acids that constitute the protein of the present invention and the remainder, and if the purified product has a protective group, removing the protective group. Known methods of condensation and the methods of protective group removal described below, for example, can be used.
(1) M. Bodanszky and M.A. Ondetti, Peptide Synthesis, Interscience Publishers, New York (1966)
(2) Schroeder and Luebke, The peptide, Academic Press, New York (1965)
(3) Nobuo Izumiya et al., *Peputido Dogei no Kiso to Jikken,* Maruzen Co., Ltd. (1975)
(4) Haruaki Yajima and Shunpei Sakakibara, *Seikagaku Jikken Kouza* 1, *Tanpakushitsu no Kagaku* IV, 205, (1977)
(5) Haruaki Yajima, supervisor, *Zoku Iyakuhin no Kaihatsu,* Vol. 14: Peputido Gosei, Hirokawa Publishing

After the reaction, the protein of the present invention can be purified and isolated using ordinary purification methods such as solvent extraction, distillation, column chromatography, liquid chromatography, and recrystallization in combination. If the protein obtained by the method described above is a free from, it can be converted to an appropriate salt by a known method; if the protein obtained is a salt, it can be converted to a free form by a known method.

The thus-obtained antibody, complex and/or protein (anti-environmental hormone antibody recombinant protein) of the present invention can be used as a reagent for quantitatively measuring environmental hormones, or can be used to produce affinity columns for concentrating environmental hormones, in which it is immobilized to various carriers. Additionally, by identifying an environmental hormone that binds (i.e., cross-reacts) to the antibody, complex and/or protein of the present invention, the applicability of the antibody, complex and/or protein of the present invention can be expanded. Furthermore, the present invention provides a kit for measuring or quantifying an environmental hormone containing the antibody, complex and/or protein of the present invention and a kit for concentrating an environmental hormone.

Although the aforementioned kit may contain only one kind of the antibody, complex and/or protein of the present invention, it may also contain plural kinds of antibody, complex and/or protein of the present invention. For example, by using a kit containing a plurality of complexes of different degrees of cross-reactivity, a particular environmental hormone (e.g., NP10EO, NP1EC, NP) can be determined or quantified with specificity. Specifically, to specifically measure and quantify NP in a sample containing a plurality of environmental hormones such as NP10EO, NP1EC, NP and the like, a complex that binds to NP and another complex that does not substantially bind to NP may be used in combination (it is preferable, however, that these complexes be similar in terms of cross-reactivity with the other environmental hormones contained in the sample). The present invention also provides such a method.

As methods of measuring environmental hormones using the antibody, complex and/or protein of the present invention, there may be mentioned various methods in common use for antigen detection, such as radioisotope immunoassay (RIA), ELISA [Engvall, E., Methods in Enzymol., 70, 419-439 (1980)], fluorescent antibody method, plaque method, spot method, agglutination method, and Ouchterlony method ("Hybridoma Method and Monoclonal Antibodies", published by R&D Planning, pages 30-53, March 5, 1982). From the viewpoint of sensitivity, simplicity, etc., ELISA is commonly used.

As a carrier for the antibody and/or protein of the present invention, there may be mentioned, for example, microplates (e.g., 96-well microplate, 24-well microplate, 192-well microplate, 384-well microplate, etc.), test tubes (e.g., glass test tubes, plastic test tubes), glass particles, polystyrene particles, modified polystyrene particles, polyvinyl particles, latexes (e.g., polystyrene latex), nitrocellulose membranes, cyanogen bromide-activated filter paper, DBM-activated filter paper, granular solid phases (e.g., Sepharose, Sephadex, agarose, cellulose, Sephacryl, etc.), iron-containing polycarbonate films, and magnet-containing beads.

The antibody, complex and/or protein of the present invention can be carried on carriers by methods known *per se* [e.g., "Enzyme Immunoassay" above, pp. 268~296, "Affinity Chromatography Handbook", Amersham-Pharmacia Biotech K.K., published December 20, 1998].

In the immunological concentration method of the present invention, the desired substance of minimal immunological impurity contents can be concentrated at rates as high as several thousands to several tens of thousands of times, by passing a large amount of sample through a column of immunological adsorbent or mixing with immunological adsorbent particles to adsorb the desired environmental hormone, degradation products thereof or a mixture thereof to the immunological adsorbent by an antigen-antibody reaction, and subsequently eluting the desired product by known methods, such as changing the pH (lowering to pH 2.5~3, raising to pH 11.5), changing the ionic strength (1M NaCl etc.), changing the polarity [10% dioxane, 50% ethylene glycol, 3M chaotropic salts (SCN⁻, CCl₃COO⁻, I⁻), etc.], adding protein denaturants (8M urea, 6M guanidine hydrochloride, etc.), and conducting electrophoretic dissociation.

Environmental hormones occurring in very trace amounts in the environment, degradation products thereof, or a mixture thereof can thereby be concentrated at much higher rates than by conventional methods of concentration such as solvent extraction and solid phase extraction and in addition concentrates of lower contents of impurities and other substances that interfere with quantitation can be obtained.

When bases, amino acids and the like are shown in abbreviations in the present specification and Figures, they are based on the abbreviations by IUPAC-IUB Commission on Biochemical Nomenclature or abbreviations conventionally used in the pertinent field. Examples thereof are given in the following. When an amino acid can have an optical isomer, it is an L form, unless particularly indicated.
DNA :deoxyribonucleic acid
cDNA :complementary deoxyribonucleic acid
a,A :Adenine
t,T :Thymine
g,G :Guanine
c,C :Cytosine
RNA :ribonucleic acid
mRNA :messenger ribonucleic acid
Abbreviations of amino acids
3 letters:1 letter:Japanese name
Gly : G :glycine
Ala : A :alanine
Val : V :valine
Leu : L :leucine
Ile : I :isoleucine
Ser : S :serine
Thr : T :threonine
Cys : C :cysteine
Met : M :methionine
Glu : E glutamic acid
Asp : D :aspartic acid
Lys : K :lysine
Arg : R :arginine
His : H :histidine
Phe : F :phenylalanine
Tyr : Y :tyrosine
Trp : W :tryptophan
Pro : P :proline
Asn : N :asparagine
Gln : Q :glutamine
Asx : B :Asn+Asp
Glx : Z :Gln+Glu

### Examples

The present invention is explained in more detail by illustrating Examples in the following, which are not to be construed as limitative.

The Escherichia coli transformant produced in the following Example 6 have been deposited at National Institute of Advanced Industrial Science and Technology, International Patent Organism Depository, Central 6, 1-1-1 Higashi, Tsukubashi, Ibaraki, Japan under the Budapest Treaty. Their deposit numbers and the date of deposit are shown in the following.
(1) Escherichia coli BL/pET-APscFvHL:
   Deposit number: FERM BP-7910
   Date of deposit: February 22, 2002
(2) Escherichia coli BL/pET-MOFscFvHL:
   Deposit number: FERM BP-7911
   Date of deposit: February 22, 2002

In the following, the abbreviations shown below are used as necessary.
AP-14VH: heavy chain variable region of anti-alkylphenol monoclonal antibody
AP-14VL: light chain variable region of anti-alkylphenol monoclonal antibody
AP-14scFv: single chain variable fragment antibody (comprising AP-14scFvHL and AP-14scFvLH described below) comprising AP-14VH and AP-14VL
AP-14scFvHL: single chain variable fragment antibody comprising AP-14VH at N terminal and AP-14VL at C terminal
AP-14scFvLH: single chain variable fragment antibody comprising AP-14VL at N terminal and AP-14VH at C terminal
AP-14scFvVH: heavy chain variable region of AP-14scFv
AP-14scFvVL: light chain variable region of AP-14scFv
MOF3-139VH: heavy chain variable region of anti-alkylphenol ethoxylate monoclonal antibody
MOF3-139VL: light chain variable region of anti-alkylphenol ethoxylate monoclonal antibody
MOF3-139scFv: single chain variable fragment antibody (comprising MOF3-139scFvHL and MOF3-139scFvLH described below) comprising MOF3-139 VH and MOF3-139 VL
MOF3-139scFvHL: single chain variable fragment antibody comprising MOF3-139 VH at N terminal and MOF3-139 VL at C terminal
MOF3-139scFvLH: single chain variable fragment antibody comprising MOF3-139 VL at N terminal and MOF3-139 VH at C terminal
MOF3-139scFvVH: heavy chain variable region of MOF3-139scFv
MOF3-139scFvVL: light chain variable region of MOF3-139scFv

### [Example 1] Cloning of cDNA encoding heavy chain and light chain of an anti-alkylphenol monoclonal antibody and an anti-alkylphenolethoxyate monoclonal antibody using 5'-Rapid Amplification of cDNA Ends (RACE) method

Mouse hybridoma cell AP-14 (FERM BP-6633) producing an anti-alkylphenol monoclonal antibody, and mouse hybridoma cell MOF3-139 (FERM BP-6059) producing an anti-alkylphenol ethoxylate monoclonal antibody (they were produced by the method described in Examples of PCT international publication WO99/43799) were used as starting materials. Firstly, about 2 µg of mRNAs were respectively extracted from 2x10⁷ of each hybridoma cells cultured in DMEM medium (purchased from ICN) containing 10% fetal calf serum (purchased form JRH) using QuickPrep Micro mRNA Purification Kit (purchased from Amersham Pharmacia Biotech) according to attached instructions. Using these mRNAs as templates and SMART-RACE cDNA Amplification Kit (purchased from Clontech) according to attached instructions, reverse transcription reactions were carried out to synthesize single strand cDNAs respectively. Since it was identified that subclass of AP-14 was IgG2b and κ chain and subclass of MOF3-139 was IgG1 and κ chain, Cgamma1 primer specific for constant region of heavy chain IgG1, Cgamma2b primer specific for constant region of heavy chain IgG2b, and Ckappa primer specific for constant region of light chain were synthesized. cDNAs encoding the light chain and the heavy chain of AP-14 and MOF3-139 were respectively isolated by 5'-RACE method using these three kinds of primers and SMART-RACE cDNA Amplification Kit.

Cgamma1 primer :
5′-GACAGATGGGGGTGTCGTTTTGGC-3′(SEQ ID NO:9)

Cgamma2b primer :
5′-GGAGGAACCAGTTGTATCTCCACACC-3′ (SEQ ID NO:10)

Ckappa primer :
5′-AGATGGATACAGTTGGTGCAGCATCAGC-3′(SEQ ID NO:11)

Composition of PCR reaction mixture for amplifying RACE product was 34.5 µL of sterilized water, 5 µL of 10 X Advantage 2 PCR Buffer (attached with SMART-RACE cDNA Amplification Kit), 1 µL of 10 mM dNTP Mix (purchased from Clontech), 1 µL of 50 X Advantage 2 Polymerase Mix (attached with SMART-RACE cDNA Amplification Kit), 2.5 µL of cDNA samples and 5 µL of Universal Primer Mix (attached with SMART-RACE cDNA Amplification Kit), as well as 1 µL of 10 µM Cgamma1 primers, 10 µM Cgamma2b primers or 10 µM Ckappa primers in 50 µL of total volume. In PCR, reaction of 30 seconds at 94°C, 30 seconds at 68°C and 3 minutes at 72°C was repeated 30 times. The reaction solutions were electrophoresed on 1.5% agarose gel. As a result, about 600 bp of cDNA fragment deduced to comprise a portion encoding the variable region of the light chain, and about 600 bp of cDNA fragment deduced to comprise a portion encoding the variable region of the heavy chain were amplified.

### [Example 2] Determination of base sequence of an anti-alkylphenolmonoclonal antibody and an anti-alkylphenol ethoxylate monoclonal antibody

cDNA fragments encoding the variable regions of the heavy chains and the light chains prepared in Example 1 were inserted into cloning vector pT7Blue T-vector (purchased from Novagen), and introduced to Escherichia coli JM109. Recombinant pT7Blue T-vectors were extracted from grown Escherichia coli, base sequences of the variable regions of the heavy chains and the light chains of AP-14 and MOF3-139 were determined with dideoxy method. The base sequences are shown as SEQ ID NO:1, SEQ ID NO:3, SEQ ID NO:5 and SEQ ID NO:7. Amino acid sequences corresponding to them are shown as SEQ ID NO:2, SEQ ID NO:4, SEQ ID NO:6 and SEQ ID NO:8.

Specifically, SEQ ID NO:1 shows base sequence of heavy chain variable region gene of antibody produced by mouse hybridoma cell line AP-14, and SEQ ID NO:2 shows amino acid sequence corresponding thereto.

SEQ ID NO:3 shows base sequence of light chain variable region gene of antibody produced by mouse hybridoma cell line AP-14, and SEQ ID NO:4 shows amino acid sequence corresponding thereto.

SEQ ID NO:5 shows base sequence of heavy chain variable region gene of antibody produced by mouse hybridoma cell line MOF3-139, and SEQ ID NO:6 shows amino acid sequence corresponding thereto.

SEQ ID NO:7 shows base sequence of light chain variable region gene of antibody produced by mouse hybridoma cell line MOF3-139, and SEQ ID NO:8 shows amino acid sequence corresponding thereto.

### [Example 3] Synthesis of primers

Using base sequence information of the variable regions of the heavy chains and the light chains of AP-14 and MOF3-139 determined in Example 2, 12 kinds of primers indicated below were synthesized for constructing AP-14scFv and MOF3-139scFv genes and chimera gene.

primer 01 (sequence length: 23)
5'-CCATGGATCAGGTCCAGCTCCAGCAGTCT-3' (SEQ ID NO:12)

primer 02 (chain length: 52)
5'-AGAGCCACCTCCGCCTGAACCGCCTCCACCTGAGGAGACGGTGACTGAGGTT-3'

(SEQ ID NO:13)
primer 03 (chain length: 52)
5'-GGCGGAGGTGGCTCTGGCGGTGGCGGATCGGATATTGTGATGACGCAGGCTG -3'
(SEQ ID NO:14)

primer 04 (chain length: 29)
5'-GCTAGCCCGTTTTATTTCCAGCTTGGTC-3' (SEQ ID NO:15)

primer 05 (chain length: 28)
5'-CCATGGATCAGGTTCAGCTGCAGCAGTCT-3'(SEQ ID NO:16)

primer 06 (chain length: 53)
5'-AGAGCCACCTCCGCCTGAACCGCCTCCACCTGAGGAGACTGTGAGAGTGGTGC-3'
(SEQ ID NO: 17)

primer 07 (chain length: 30)
5′-CCATGGATGATATTGTGATGACGCAGGCTG-3′ (SEQ ID NO:18)

primer 08 (chain length:52)
5'-AGAGCCACCTCCGCCTGAACCGCCTCCACCCCGTTTATTTCCAGCTTGGTC-3'
(SEQ ID NO:19)

primer 09 (chain length: 51)
5′-GGCGGAGGTGGCTCTGGCGGTGGCGGATCGCAGGTCCAGCTCCAGCAGTCT-3′ (SEQ
ID NO: 20)

primer 10 (chain length: 28)
5′-GCTAGCTGAGGAGACGGTGACTGAGGTT-3′ (SEQ ID NO:21)

primer 11 (chain length: 51)
5'-GGCGGAGGTGGCTCTGGCGGTGGCGGATCGCAGGTCCAGCTCCAGCAGTCT-3' (SEQ
ID NO: 22)

primer 12 (chain length: 29)
5′-GCTAGCTGAGGAGACTGTGAGAGTGGTGC-3′ (SEQ ID NO:23)

### [Example 4] Construction of single chain variable fragment antibody (scFv) gene of an anti-alkylphenol monoclonal antibody and an anti-alkylphenolethoxylate monoclonal antibody

### 1) Construction of AP-14scFvHL gene

AP-14scFvHL gene was constructed using primer 01 and primer 02 for amplifying AP-14 VH gene, primer 03 and primer 04 for amplifying AP-14 VL gene and primer 01 and primer 04 for constructing its scFv gene in accordance with following procedures.

### 2) Construction of MOF3-139scFvHL gene

MOF3-139scFvHL gene was constructed using primer 05 and primer 06 for amplifying MOF3-139 VH gene, primer 03 and primer 04 for amplifying MOF3-139 VL gene and primer 05 and primer 04 for amplifying its scFv gene in accordance with following procedures.

### 3) Construction of AP-14scFvLH gene

AP-14scFvLH gene was constructed using primer 07 and primer 08 for amplifying AP-14 VL gene, primer 09 and primer 10 for amplifying AP-14 VH gene and primer 07 and primer 10 for amplifying its scFv gene in accordance with following procedures.

### 4) Construction of MOF3-139scFvLH gene

MOF3-139scFvLH gene was constructed using primer 07 and primer 08 for amplifying MOF3-139VL gene, using primer 11 and primer 12 for amplifying MOF3-139 VH gene and primer 07 and primer 12 for amplifying its scFv gene in accordance with following procedures.

### 5) Construction of AP-14VH/MOF3-139VL gene

AP-14VH/MOF3-139VL gene was constructed using primer 01 and primer 02 for amplifying AP-14 VH gene, primer 03 and primer 04 for amplifying MOF3-139VL gene and primer 01 and primer 04 for amplifying its scFv gene in accordance with following procedures.

### 6) Construction of MOF3-139VH/AP-14VL gene

AP-14VH/MOF3-139VL gene was constructed using primer 05 and primer 06 for amplifying MOF3-139 VH gene, primer 03 and primer 04 for amplifying AP-14VL gene and primer 05 and primer 04 for amplifying its scFv gene in accordance with following procedures.

### 7) Construction of AP-14VL/MOF3-139VH gene

AP-14VL/MOF3-139VH gene was constructed using primer 07 and primer 08 for amplifying AP-14VL gene, primer 11 and primer 12 for amplifying MOF3-139 VH gene and primer 07 and primer 12 for amplifying its scFv gene in accordance with following procedures.

### 8) Construction of MOF3-139VL/AP-14VH gene

MOF3-139VL/AP-14VH gene was constructed using primer 07 and primer 08 for amplifying MOF3-139VL gene, primer 09 and primer 10 for amplifying AP-14 VH gene and primer 07 and primer 10 for amplifying its scFv gene in accordance with following procedures.

The genes were constructed by PCR. Firstly, PCR reactions were carried out using plasmid inserted with cDNA encoding the variable regions of the heavy chain or the light chain of AP-14 and MOF3-139, and primers corresponding to each plasmid in order to amplify cDNA fragments encoding the variable regions of the heavy chain and the light chain. For example, composition of PCR reaction mixture was 1 µL of 50 X Advantage cDNA Polymerase Mix (purchased from Clontech), 5 µL of 10 X cDNA PCR Reaction Buffer (purchased from Clontech), 1 µL of 50 X dNTP Mix (purchased from Clontech), 0.5 µL of recombinant plasmid (in this case, recombinant pT7Blue T-vector inserted with cDNA fragment encoding the variable region of the heavy chain of AP-14)(100 ng), 0.5 µL of primer 01 (50 pmol), 0.5 µL of primer 02 (50 pmol) and 41.5µL of sterilized water in 50 µL of the total volume, when the heavy chain of AP-14 was amplified. Reaction was repeated 30 times under PCR condition of 30 seconds at 94°C, 30 seconds at 63°C and 1 minute at 72°C. cDNAs encoding the heavy chain and the light chain of AP-14 and MOF3-139 were amplified respectively, then reaction solution were electrophoresed on 1.5% agarose gel. As a result, amplification of about 400 bp of cDNA fragment encoding the variable region of the light chain, and about 400 bp of cDNA fragment encoding the variable region of the heavy chain was confirmed respectively. cDNA fragments were purified respectively, and then used for constructing scFv gene in which the variable regions of the heavy chain and the light chain is linked. PCR reaction was carried out using cDNA fragments encoding the variable regions of the heavy chain and the light chain. For example, composition of PCR reaction mixture was 21.5 µL of lysis buffer for cDNA encoding the variable region of the heavy chain of AP-14, 21.5 µL of lysis buffer for cDNA encoding the variable region of the light chain of AP-14, 5 µL of 10 X cDNA PCR Reaction Buffer, 1 µL of 50 X d NTP Mix, 1 µL of 50 X Advantage cDNA Polymerase Mix in 50 µL of total volume, when the heavy chain of AP-14 was amplified. Reaction was repeated 7 times under PCR condition of 30 seconds at 94°C, 30 seconds at 55°C and 1 minute at 72°C. Thereafter, 1 µL of 50 X Advantage cDNA Polymerase Mix, 5 µL of 10 X cDNA PCR Reaction Buffer, 1 µL of 50 X dNTP Mix, 0.5 µL of primer 01 (50 pmol), 0.5 µL of primer 06 (50 pmol) and 42 µL of sterilized water was used to 50 µL of total amount, and added to the aforementioned PCR reaction mixture. Reaction was repeated 30 times under PCR condition of 30 seconds at 94°C, 30 seconds at 63°C and 1 minute at 72°C. The reaction solution was electrophoresed on 1.5% agarose gel, amplification of about 750 bp of AP-14scFv and MOF3-139scFv gene fragment was confirmed, and purified.

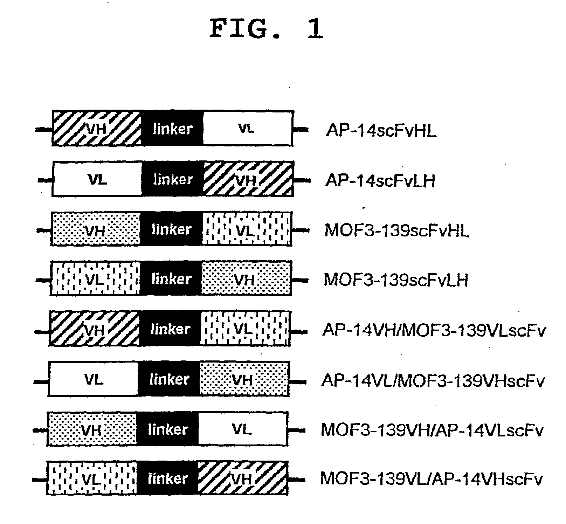

A summary of 8 kinds of anti-environmental hormone single chain variable region antibodies prepared by the following procedures is shown in Fig. 1.

### [Example 5] Construction of scFv expression plasmids of anti-alkylphenol monoclonal antibody and anti-alkylphenol ethoxylate monoclonal antibody

The eight kinds of genes prepared in Example 4 were each inserted in cloning vector pT7 Blue T-vector to transform Escherichia coli JM109 strain. The introduced recombinant plasmid was extracted from the grown Escherichia coli, and the base sequence of the gene was confirmed. The recombinant plasmid in which the gene had been inserted and the Escherichia coli expression vector pET-27b were digested using restriction enzymes *Nco*I and *Nhe*I respectively, and the gene and the Escherichia coli expression vector pET-27b was ligated to construct the following expression plasmids
pET-27b/AP-14scFvHL,
pET-27b/AP-14scFvLH,
pET-27b/MOF3-139scFvHL,
pET-27b/MOF3-139scFvLH,
pET-27b/AP-14VH/MOF3-139VLscFv,
pET-27b/MOF3-139VL/AP-14VHscFv,
pET-27b/MOF3-139VH/AP-14VLscFv and
pET-27b/AP-14VL/MOF3-139VHscFv.

### [Example 6] Preparation of scFvs of anti-alkylphenol monoclonal antibody and anti-alkylphenol ethoxylate monoclonal antibody

The eight kinds of expression plasmids prepared in Example 5 were each introduced in Escherichia coli BL21DE3 (pLysS) strain to produce eight strains of transformant in which an expression plasmid had been introduced.

A strain in which AP-14scFvHL expression plasmid pET-27b/AP-14 scFvHL has been introduced in Escherichia coli BL21DE3 (pLysS) strain is referred to as Escherichia coli BL/pET-APscFvHL, which has the deposit number of FERM BP-7910, and a strain in which MOF3-139scFvHL expression plasmid pET-27b/MOF3-139scFvHL has been introduced in Escherichia coli BL21DE3 (pLysS) strain is referred to as Escherichia coli BL/pET-MOFscFvHL, which has the deposit number of FERM BP-7911. Both were deposited at the National Institute of Advanced Industrial Science and Technology, International Patent Organism Depositary.

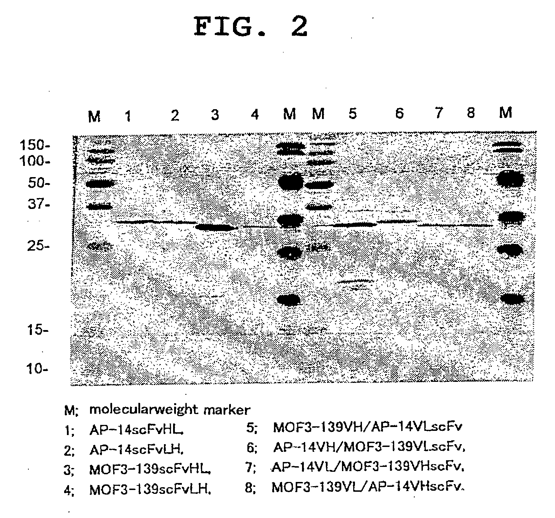

The eight kinds of transformed colonies obtained as above were each seeded on 2 X YT medium (5 mL, comprising 1.7% (w/v) of Bacto-trypton (purchased from Nacalai Tesque, Inc.), 1% (w/v) of Bacto-yeast extract (purchased from Nacalai Tesque, Inc.) and 0.5% of (w/v) NaCl (purchased from Nacalai Tesque, Inc.)) comprising 25 µg/mL of kanamycin (purchased from Nacalai Tesque, Inc.) and 33 µg/mL of chloramphenicol (purchased from Nacalai Tesque, Inc.) and cultured at 23°C overnight with shaking. The suspension was suspended in SB medium (800 mL, 3.5% (w/v) of Bacto-trypton, 2% (w/v) of Bacto-yeast extract and 0.5% (w/v) of NaCl) at 23°C overnight with shaking. To the suspension was added isopropylthiogalactoside (IPTG) (purchased from Nacalai Tesque, Inc.) so that the final concentration became 1 mM and further cultured at 23°C for 6 hr with shaking. The suspension was separated by centrifugation at 1500 g for 20 min and the supernatant was removed. The precipitated fungus body was suspended in ice-cooled TES buffer (32 mL, comprising 0.2 M Tris-HCl (pH 8.0), 0.5 mM EDTA and 0.5 M sucrose (each was purchased from Nacalai Tesque, Inc.)). To the suspension was further added ice-cooled 0.2X TES buffer (48 mL, comprising 0.04 M Tris-HCl (pH 8.0), 0.1 mM EDTA and 0.1 M sucrose) and stirred sufficiently. The suspension was cooled with ice for 30 min and separated by centrifugation at 12000 g for 10 min, and the obtained supernatant was used for AP-14scFv and MOF3-139scFv. Expression of the desired protein was confirmed by Western Blot method using an anti-HSV tagged antibody (mentioned below). The results are shown in Fig. 2. It was confirmed that eight kinds of scFv antibodies (about 30 kDa) were each expressed in *Escherichia coli.*

### Western Blot method:

About 10 µg of scFv was subjected to 10% dodecyl sodium sulfate-polyacrylamide electrophoresis (SDS-PAGE) and the scFv in the gel was transferred to a polyvinylidene difluoride (PVDF) membrane. Blocking was carried out using Block Ace, and the PVDF membrane was soaked in a solution of anti-HSV antibody (0.1 µg/mL) and shaken at room temperature for 1 hr. The PVDF membrane was washed, soaked in an antibody solution comprising alkaline phosphatase-labeled anti-mouse antibody and shaken at room temperature for 30 min. The PVDF membrane was washed, and coloring reaction was carried out by soaking the membrane in a solution of a chromogen substrate (nitroblue tetrazolium/5-bromo-4-chloro-3-indolylphosphoric acid).

### [Example 7] Comparison of performance of antibody by indirect competitive ELISA

A conjugate (5 µg/mL) of hapten and bovine serum albumin (BSA) (100 µL/each well) was added to a 96-well microtiter plate and stood at 4°C overnight. After removing the liquid from the well, 300 µL of Block Ace (purchased from Dainippon Pharmaceutical Co., Ltd.) 4-fold diluted with distillated water was added to each well and stood at 4°C overnight. After removing the liquid from the well, a standard solution (50 µL) of nonylphenol decaethoxylate stepwise diluted with phosphate buffer (10 mM sodium phosphate (purchased from Nacalai Tesque, Inc.), 150 mM NaCl, pH 7.2) was added to each well. AP-14scFv or MOF3-139scFv prepared in Example 6 was diluted with a phosphate buffer and the solution (50 µL) was added to each well. The mixture was incubated at 25°C for 1 hr and each well was washed 4 times with a phosphate buffer. Then, 100 µL of mouse anti-HSV tagged antibody (purchased from Amersham Pharmacia Biotech), diluted with a phosphate buffer to 0.1 µg/mL, was added to each well and the mixture was incubated at 25°C for 1 hr. After washing each well 4 times with a phosphate buffer, 100 µL of a peroxydase-labeled goat antibody against mouse antibody (purchased from Pierce), 2,000-fold diluted with a phosphate buffer, was added to each well and the mixture was incubated at 25°C for 1 hr. After washing each well 3 times with a phosphate buffer, 100 µL of chromogeic substrate solution (100 mg/L tetramethylbenzidine (purchased from Wako Pure Chemical Industries, Ltd.) and 0.006% (v/v) aqueous hydrogen peroxide solution were added into 0.1 M acetate buffer (pH 5.5)) was added to each well. The mixture was incubated at room temperature for 10 min. Then, 1 N sulfuric acid (50 µL/well) was added to stop the enzyme reaction. The absorbance at wavelength of 450 nm was measured.

As a control, indirect competitive ELISA was conducted by using a monoclonal antibody from hybridoma. As described above, a standard solution (50 µL/each well) of nonylphenol decaethoxylate, serially diluted with a phosphate buffer, was added to a 96-well microtiter plate having a conjugate of hapten and BSA fixed thereto. AP-14 or MOF3-139 was diluted with a phosphate buffer, and the solution (50 µL/well) was added to each well. The mixture was incubated at 25°C for 1 hr and each well was washed 3 times with a phosphate buffer. Then, 100 µL of a peroxydase-labeled goat antibody against mouse antibody (purchased from Pierce) 2,000-fold diluted with a phosphate buffer was added to each well and the mixture was incubated at 25°C for 1 hr. After washing each well 3 times with a phosphate buffer, 100 µL of the above-mentioned chromogeic substrate solution was added to each well. The mixture was incubated at room temperature for 10 min. Then, 1N sulfuric acid (50 µL/well) was added to stop the enzyme reaction. The absorbance at wavelength of 450 nm was measured.

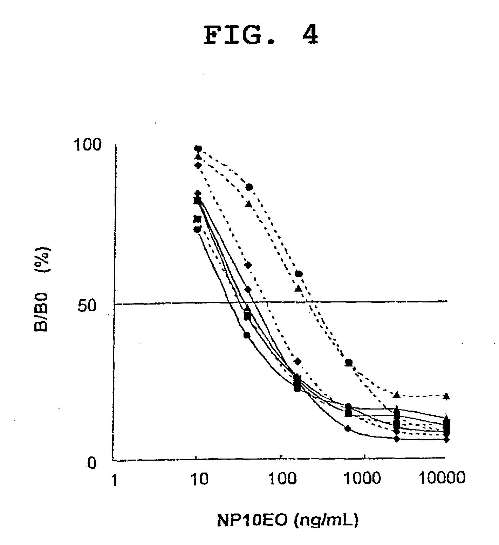

The results are shown in Fig. 4. As is clear from Fig. 4, an anti-environment hormone single chain variable fragment antibody obtained in the present invention can be used for a quantitative determination by ELISA method. In addition, from the results of control test conducted by indirect competitive ELISA using a monoclonal antibody from hybridoma, it was found that the anti-environment hormone single chain variable fragment antibody had reactivity similar to that of the original monoclonal antibody. Accordingly, by using the anti-environment hormone single chain variable fragment antibody of the present invention, a sequence of single chain variable region can be optionally changed, chimera genes of antibody genes obtained from different hybridoma can be produced, amino acid sequence at random site can be modified and an antibody highly resistant to various inhibitors, solvents and the like requested for the measurement by an immunity measurement method can be artificially made.

### [Example 8] Measurement of cross-reactivity of scFv with various antigens

Then, cross reactivity of scFvs with various antigens was measured by indirect competitive ELISA. For comparison, cross reactivity of anti-alkylphenol monoclonal antibody and that of anti-alkylphenol ethoxylate monoclonal antibody with various antigens were also measured. The indirect competitive ELISA was conducted by a method substantially similar to the method in Example 7. The cross-reactivity was calculated by dividing IC₅₀ value of NP10EO by the value of IC₅₀ of test compound and multiplying by 100. The results are shown in Fig. 5.

As a result, it was found that antigen specificities of scFv were similar (Fig. 5). However, it was found that the cross reactivity with NP1EC and that with NP were clearly different. In particular, anti-alkylphenol monoclonal antibody and scFv having AP-14scFvVH shows cross-reactivity with NP1EC and NP, while anti-alkylphenolethoxylate monoclonal antibody and scFv having MOF3-139scFvVH hardly shows cross-reactivity with NP1EC and NP.

As described above, it was suggested that a heavy chain variable region (AP-14scFvVH and MOF3-139scFvVH) of scFv concerns the difference of cross-reactivity with NP1EC and NP.

### [Example 9] Identification of the region in AP-14scFvVH involved in reactivity with NP1EC and NP

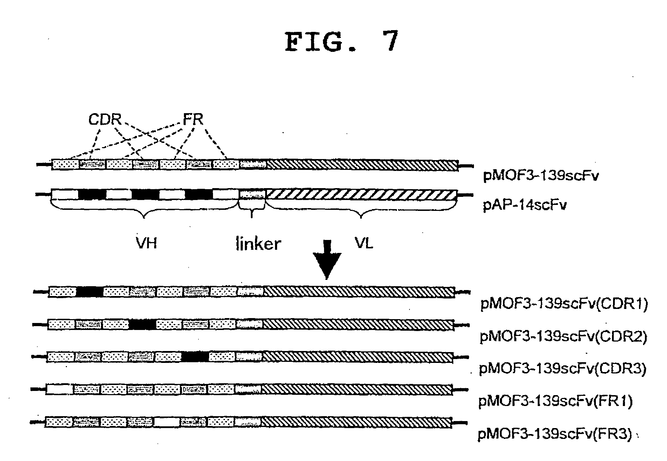

Next, in order to identify the region in AP-14scFvVH involved in reactivity with NP1EC and NP, comparison between amino acid sequences of variable region of AP-14scFv and MOF3-139scFv was performed (Fig. 6). As a result, the amino acid sequence of AP-14scFvVL was different from the sequence of MOF3-139scFvVL in only 5 amino acid residues. In contrast, the amino acid sequence of AP-14scFvVH was extremely different from the amino acid sequence of MOF3-139scFvVH. Particularly, relatively many differences could be found in the complementarity determining region (CDR) 1, 2 and 3 and framework region (FR) 1 and 3. Then, in order to make a detailed analysis as to which region mainly is involved in the difference in the reactivity with NP1EC and NP, MOF3-139scFv mutant gene was constructed by incorporating CDR1, CDR2, CDR3, FR1 and FR3 in AP-14scFvVH into the corresponding regions in MOF3-139scFvVH, respectively (Fig. 7) and expressed in *Escherichia coli* by the method substantially the same as that described in Example 6. Then, the obtained MOF3-139scFv mutants (MOF3-139scFv(CDR1), MOF3-139scFv(CDR2), MOF3-139scFv(CDR3), MOF3-139scFv(FR1) and MOF3-139scFv(FR3)) were subjected to indirect competitive ELISA, and reactivity with NP10EO, NP1EC and NP was measured. Here, indirect competitive ELISA was conducted by the method substantially the same as that described in Example 7. The results are indicated in the relative reactivity (%), which is calculated by dividing IC₅₀ value of AP-14scFv against NP10EO by IC₅₀ value of MOF3-139scFv mutant or wild MOF3-139scFv against NP10EO and multiplying the value by 100.

As a result, reactivity of MOF3-139scFv(CDR1) with NP10EO decreased, whereas reactivity with NP10EO of other MOF3-139scFv mutants having mutation in CDR and FR scarcely changed (Fig. 8 (A)). In addition, reactivity of MOF3-139scFv(CDR1) with NP1EC, and reactivity of MOF3-139scFv(CDR2) with NP remarkably increased respectively (Figs. 8(B) and 8(C)).

From the above, it is suggested that CDR1 in AP-14scFvVH is involved in reactivity with NP1EC, and CDR2 in AP-14scFvVH is involved in reactivity with NP.

### [Example 10] Identification of amino acid residue in AP-14scFvVH involved in reactivity with NP1EC and NP

In order to make a detailed analysis of reactivity of AP-14scFv with NP1EC and NP, reactivity of MOF3-139scFv mutant with NP1EC and NP, wherein one amino acid residue was mutated, was examined. First, CDR1 and CDR2 in AP-14scFvVH and MOF3-139scFvVH were compared at the amino acid level. As a result, it has been revealed that the amino acid sequences at CDR1 between AP-14scFvVH and MOF3-139scFvVH were different in 6 residues, whereas the amino acid sequence at CDR2 was different in 5 residues. Then, MOF3-139scFv mutant was prepared, wherein amino acid of AP-14scFv was substituted by amino acid of MOF3-139Fv with regard to 11 amino acid residues found to be different in the amino acid sequences at CDR1 and CDR2 (Table 1).

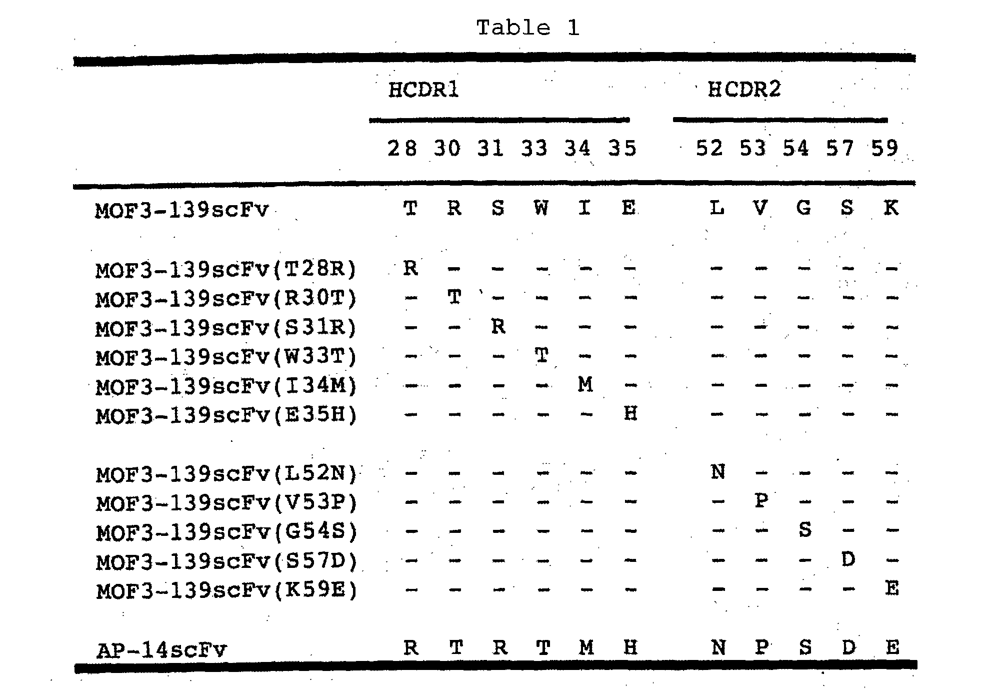

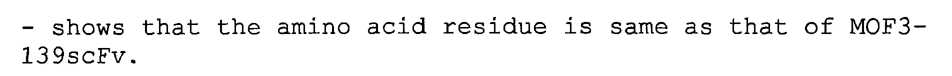

- shows that the amino acid residue is same as that of MOF3-139scFv.

Then, in the same manner as in Example 9, the reactivity with NP10EO was measured. As a result, MOF3-139scFv mutant wherein the 30th arginine was substituted by threonine and MOF3-139scFv mutant wherein the 57th serine was substituted by aspartic acid showed increased reactivity with NP10EO (Fig. 9(A), 9(B)). In addition, MOF3-139scFv mutant wherein the 28th threonine was substituted by arginine and the 34th isoleucine was substituted by methionine showed nearly 10-fold decreased reactivity with NP10EO. Other MOF3-139scFv mutants showed almost the same as or slightly decreased reactivity with NP10EO,

Subsequently, the reactivity with NP1EC was measured. As a result, MOF3-139scFv mutant wherein the 33rd tryptophan was substituted by threonine and the 35th glutamic acid was substituted by histidine showed 8 to 9-fold increased reactivity with NP1EC as compared to wild type MOF3-139scFv (Fig. 9(C), 9(D)). In the former case, the increased reactivity is considered to be attributable to interaction (hydrogen bond etc.) with the adjacent amino acid residue, which was caused by the substitution to a neutral amino acid, and in the latter case, the increased reactivity is assumed to be attributable to a direct interaction of positive charged amino acid with carboxylic acid of NP1EC, which was caused by substituting negative charged amino acid by a positive charged amino acid. Since involvement of these two amino acid residues of AP-14 in the binding to NP1EC was suggested, narrowing down of amino acid residues using the CDR-substituted scFv mutant is considered to have been effective.

Moreover, the reactivity with NP was measured in the same manner. As a result, 6 kinds of scFv mutants showed 3 to 6-fold increased reactivity with NP as compared to the reactivity of MOF3-139scFv (Fig. 9(E), 9(F)). Particularly, MOF3-139scFv mutant wherein the 33rd tryptophan was substituted by threonine, the 57th serine was substituted by aspartic acid, and the 59th lysine was substituted by glutamic acid showed the same level of reactivity as did AP-14scFv. The former was also involved in the reactivity with NP1EC and the latter two kinds interestingly showed an increased reactivity due to the substitution by acidic amino acid. Since scFv mutant comprising substituted CDR1 showed a slight increase in the reactivity with NP, it is suggested that the bond between AP-14scFv and NP1EC was affected solely by the strong interaction with a particular amino acid residue. In contrast, for the bond between AP-14scFv and NP, it is considered that the reactivity may increase not only due to a strong interaction with a particular amino acid residue but also formation of a hydrogen bond, and the like. In addition, an appropriate distance between the antigen and the side chain seems to be also important. As mentioned above, MOF3-139scFv mutant wherein the 28th threonine was substituted by arginine, MOF3-139scFv mutant wherein the 34th isoleucine was substituted by methionine both showed markedly decreased reactivity with NP10EO and NP1EC, but did not show much difference in the reactivity with NP. This suggests that substitution by these amino acids gives rise to a steric hindrance of the bond to NP10EO and NP1EC having a methylene oxide group, but NP having a smaller hydroxyl group does not interfere with the binding.

From the foregoing, an amino acid residue responsible for the difference in the binding of both monoclonal antibodies to NP1EC and NP has been clarified. Furthermore, specific substitution by an amino acid residue responsible for the different binding has made it possible to improve antigen binding ability and to change cross-reactivity with the antigen.

### Industrial Applicability

According to the present invention, a gene encoding a heavy chain or light chain of an anti-environmental hormone antibody is provided and the amino acid and base sequence thereof is clarified. It has become possible to genetically modify genes provided by the present invention, especially the genes encoding the variable regions of heavy chains and light chains; it has become possible to obtain proteins capable of binding to environmental hormones, and having preferred characteristics for measuring, quantifying and concentrating environmental hormones in large amounts, by expressing the thus-modified genes in host cells. Using a transformant microbial cell etc. harboring this modified antibody gene, it is possible to efficiently produce a recombinant protein. It is thereby possible to prepare an enzyme immunoassay kit and antibody affinity column of better performance at lower costs. In addition, modification of an amino acid residue in a heavy chain variable region, which is responsible for varied binding to an antigen, has made it possible to obtain a protein having a strong environmental hormone binding ability and various proteins having different levels of cross-reactivity with environmental hormones.

This application is based on a patent application No. 055670/2002 filed on March 1, 2002 in Japan, the contents of which are hereby incorporated by reference.

## Claims

1. A protein of the following [a] or [b], or a salt thereof:
[a] a protein having an amino acid sequence shown by SEQ ID No: 2, an amino acid sequence shown by SEQ ID No: 6, or an amino acid sequence substantially the same as these;
[b] a protein having an amino acid sequence shown by SEQ ID No: 4, an amino acid sequence shown by SEQ ID No: 8, or an amino acid sequence substantially the same as these.

2. A protein of any of the following [a¹]-[a³] and [b¹]-[b³], or a salt thereof:
[a¹] a protein consisting of an amino acid sequence of the following (i) or (ii), wherein a particular region is selected from the group consisting of a complementarity determining region 1, a complementarity determining region 2, a complementarity determining region 3, a framework region 1, a framework region 2, a framework region 3 and a framework region 4,
(i) an amino acid sequence wherein an amino acid sequence corresponding to not less than one particular region of the amino acid sequence shown by SEQ ID No: 2 has been exchanged for an amino acid sequence corresponding to the same kind of not less than one particular region contained in an amino acid sequence shown by SEQ ID No: 6,
(ii) an amino acid sequence wherein an amino acid sequence corresponding to not less than one particular region of the amino acid sequence shown by SEQ ID No: 6 has been exchanged for an amino acid sequence corresponding to the same kind of not less than one particular region contained in an amino acid sequence shown by SEQ ID No: 2,
[b¹] a protein having an amino acid sequence of the following
(i) or (ii), and a particular region selected from the group consisting of a complementarity determining region 1, a complementarity determining region 2, a complementarity determining region 3, a framework region 1, a framework region 2, a framework region 3 and a framework region 4,
(i) an amino acid sequence wherein an amino acid sequence corresponding to not less than one particular region of the amino acid sequence shown by SEQ ID No: 4 has been exchanged for an amino acid sequence corresponding to the same kind of not less than one particular region contained in an amino acid sequence shown by SEQ ID No: 8,
(ii) an amino acid sequence wherein an amino acid sequence corresponding to not less than one particular region of the amino acid sequence shown by SEQ ID No: 8 has been exchanged for an amino acid sequence corresponding to the same kind of not less than one particular region contained in an amino acid sequence shown by SEQ ID No: 4;
[a²] a protein having an amino acid sequence shown by SEQ ID No: 2, an amino acid sequence shown by SEQ ID No: 6 or an amino acid sequence of the protein of [a¹], wherein one or more amino acids have been deleted, substituted or added, which binds to an environmental hormone upon formation of a complex with a protein having an amino acid sequence shown by SEQ ID No: 4, an amino acid sequence shown by SEQ ID No: 8 or an amino acid sequence of the protein of [b¹];
[b²] a protein having an amino acid sequence shown by SEQ ID No: 4, an amino acid sequence shown by SEQ ID No: 8 or an amino acid sequence of the protein of [b¹], wherein one or more amino acids have been deleted, substituted or added, which binds to an environmental hormone upon formation of a complex with a protein having an amino acid sequence shown by SEQ ID No: 2, an amino acid sequence shown by SEQ ID No: 6 or an amino acid sequence of the protein of [a¹];
[a³] a protein having an amino acid sequence shown by SEQ ID No: 2, an amino acid sequence shown by SEQ ID No: 6 or an amino acid sequence of the protein of [a¹], wherein one or more amino acids have been deleted, substituted or added, which binds to an environmental hormone upon formation of a complex with a protein having an amino acid sequence shown by SEQ ID No: 4, an amino acid sequence shown by SEQ ID No: 8 or an amino acid sequence of the protein of [b¹], wherein one or more amino acids have been deleted, substituted or added;
[b³] a protein having an amino acid sequence shown by SEQ ID No: 4, an amino acid sequence shown by SEQ ID No: 8 or an amino acid sequence of the protein of [b¹], wherein one or more amino acids have been deleted, substituted or added, which binds to an environmental hormone upon formation of a complex with a protein having an amino acid sequence shown by SEQ ID No: 2, an amino acid sequence shown by SEQ ID No: 6 or an amino acid sequence of the protein of [a¹], wherein one or more amino acids have been deleted, substituted or added.

3. The protein of claim 1 or 2, or a salt thereof, wherein the environmental hormone is an alkylphenolethoxylate, an alkylphenol, a Plastic resin component, a plastic resin plasticizer or a chlorophenol.

4. The protein of claim 3, wherein the environmental hormone is an alkylphenol ethoxylate represented by the formula (1): 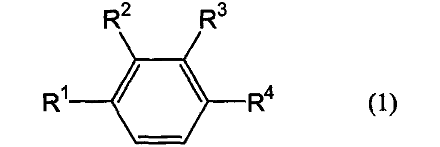 wherein R¹, R² and R³ are the same or different and each is H or a straight or branched alkyl group having 1 to 20 carbon atoms, R⁴ is (OC₂H₄)ₘOH or (OC₂H₄)ₘCOOH, and m shows an average number of ethylene oxide chain 1 to 70.

5. The protein of claim 3, wherein the environmental hormone is an alkylphenol represented by the formula (2): 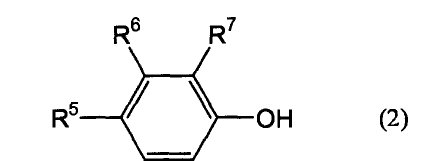 wherein R⁵, R⁶and R⁷ are the same or different and each is H or a straight or branched alkyl group having 1 to 20 carbon atoms.

6. A method of genetically recombining a protein of any of claims 1-5.

7. A protein obtained by the method of claim 6, or a salt thereof.

8. A partial peptide of a protein of any of claims 1-5 and 7, or a salt thereof.

9. A polynucleotide encoding a protein or partial peptide of a protein of any of claims 1-5 and 7.

10. A recombinant vector containing a polynucleotide of claim 9.

11. A transformant transformed with the recombinant vector of claim 10.

12. A method of producing a protein of any of claims 1-5 and 7, or a partial peptide thereof, or a salt thereof, which comprises producing a protein of any of claims 1-5 and 7, or a partial peptide thereof, or a salt thereof, and harvesting the same.

13. A complex wherein a protein of the following [a] and a protein of the following [b] are linked:
[a] a protein having an amino acid sequence shown by SEQ ID No: 2, an amino acid sequence shown by SEQ ID No: 6, or an amino acid sequence substantially the same as these;
[b] a protein having an amino acid sequence shown by SEQ ID No: 4, an amino acid sequence shown by SEQ ID No: 8, or an amino acid sequence substantially the same as these.

14. A method of identifying an environmental hormone binding to a complex of claim 13, which comprises using said complex.

15. A method of determining or quantifying an environmental hormone, which comprises using the complex of claim 13.

16. A kit for determining or quantifying an environmental hormone, which comprises the complex of claim 13.

17. A method of concentrating an environmental hormone, which comprises using the complex of claim 13.

18. A kit for concentrating an environmental hormone, which comprises the complex of claim 13.


SEQUENCE LISTING

<110>  TAKEDA CHEMICAL INDUSTRIES, LTD.
<120>  A protin binding to environmental hormones and a production thereof
<130>  09533

<150>  JP 2002-055670
<151>  2002-03-01

<160>  28

<210>  1
<211>  375
<212>  DNA
<213>  Mouse

<220>
<221>  CDS
<222>  (1)..(375)
<223>

<400>  1
cag gtc cag ctc cag cag tct gca gct gaa ctg gca aga cct ggg gcc      48
Gln Val Gln Leu Gln Gln Ser Ala Ala Glu Leu Ala Arg Pro Gly Ala
1               5                   10                  15

tca gtg aag atg tcc tgc aag gct tct ggc tat aga ttt act agg tac      96
Ser Val Lys Met Ser Cys Lys Ala Ser Gly Tyr Arg Phe Thr Arg Tyr
            20                  25                  30

acg atg cac tgg gta aaa cag agg cct gga cag ggt ctg gaa tgg att      144
Thr Met His Trp Val Lys Gln Arg Pro Gly Gln Gly Leu Glu Trp Ile
        35                  40                  45

gga aac att aat cct agc agt gga gat act gag tac aat cag aag ttc      192
Gly Asn Ile Asn Pro Ser Ser Gly Asp Thr Glu Tyr Asn Gln Lys Phe
    50                  55                  60

aag gac aag acc aca ttg act gca gac aaa tcc tcc acc aca gcc tac      240
Lys Asp Lys Thr Thr Leu Thr Ala Asp Lys Ser Ser Thr Thr Ala Tyr
65                  70                  75                  80

atg cag ctg agc agc ctg aca tct gag gac tct gcg gtc tat tac tgt      288
Met Gln Leu Ser Ser Leu Thr Ser Glu Asp Ser Ala Val Tyr Tyr Cys
                85                  90                  95

gca acc cca att ttt tac tac ggt agt agg tac gtg agg tat gaa atg      336
Ala Thr Pro Ile Phe Tyr Tyr Gly Ser Arg Tyr Val Arg Tyr Glu Met
            100                 105                 110

gac tac tgg ggt caa gga acc tca gtc acc gtc tcc tca                  375
Asp Tyr Trp Gly Gln Gly Thr Ser Val Thr Val Ser Ser
        115                 120                 125

<210>  2
<211>  125
<212>  PRT
<213>  Mouse

<400>  2
Gln Val Gln Leu Gln Gln Ser Ala Ala Glu Leu Ala Arg Pro Gly Ala
1               5                   10                  15

Ser Val Lys Met Ser Cys Lys Ala Ser Gly Tyr Arg Phe Thr Arg Tyr
            20                  25                  30

Thr Met His Trp Val Lys Gln Arg Pro Gly Gln Gly Leu Glu Trp Ile
        35                  40                  45

Gly Asn Ile Asn Pro Ser Ser Gly Asp Thr Glu Tyr Asn Gln Lys Phe
    50                  55                  60

Lys Asp Lys Thr Thr Leu Thr Ala Asp Lys Ser Ser Thr Thr Ala Tyr
65                  70                  75                  80

Met Gln Leu Ser Ser Leu Thr Ser Glu Asp Ser Ala Val Tyr Tyr Cys
                85                  90                  95

Ala Thr Pro Ile Phe Tyr Tyr Gly Ser Arg Tyr Val Arg Tyr Glu Met
            100                 105                 110

Asp Tyr Trp Gly Gln Gly Thr Ser Val Thr Val Ser Ser
        115                 120                 125

<210>  3
<211>  339
<212>  DNA
<213>  Mouse

<220>
<221>  CDS
<222>  (1)..(339)
<223>

<400>  3
gat att gtg atg acg cag gct gca ttc tcc aat cca gtc act ctt gga        48
Asp Ile Val Met Thr Gln Ala Ala Phe Ser Asn Pro Val Thr Leu Gly
1               5                   10                  15

aca tca act tcc atc tcc tgc agg tct act aag agt ctc cta cat agt        96
Thr Ser Thr Ser Ile Ser Cys Arg Ser Thr Lys Ser Leu Leu His Ser
            20                  25                  30

aat ggc atc act tat ttg tat tgg tat ctg cag aag cca ggc cag tct       144
Asn Gly Ile Thr Tyr Leu Tyr Trp Tyr Leu Gln Lys Pro Gly Gln Ser
        35                  40                  45

cct cag ctc ctg att tat cag atg tcc aac ctt gcc tca gga gtc cca       192
Pro Gln Leu Leu Ile Tyr Gln Met Ser Asn Leu Ala Ser Gly Val Pro
    50                  55                  60

gac agg ttc agt agc agt ggg tca gga act gat ttc aca ctg aga atc       240
Asp Arg Phe Ser Ser Ser Gly Ser Gly Thr Asp Phe Thr Leu Arg Ile
65                  70                  75                  80

agc aga gtg gag gct gag gat gtg ggt gtt tat tac tgt gct caa aat       288
Ser Arg Val Glu Ala Glu Asp Val Gly Val Tyr Tyr Cys Ala Gln Asn
                85                  90                  95

cta gaa ctt ccg tac acg ttc gga ggg ggg acc aag ctg gaa ata aaa       336
Leu Glu Leu Pro Tyr Thr Phe Gly Gly Gly Thr Lys Leu Glu Ile Lys
            100                 105                 110

cgg                                                                   339
Arg

<210> 4
<211> 113
<212> PRT
<213> Mouse

<400> 4
Asp Ile Val Met Thr Gln Ala Ala Phe Ser Asn Pro Val Thr Leu Gly
1               5                   10                  15

Thr Ser Thr Ser Ile Ser Cys Arg Ser Thr Lys Ser Leu Leu His Ser
            20                  25                  30

Asn Gly Ile Thr Tyr Leu Tyr Trp Tyr Leu Gln Lys Pro Gly Gln Ser
        35                  40                  45

Pro Gln Leu Leu Ile Tyr Gln Met Ser Asn Leu Ala Ser Gly Val Pro
    50                  55                  60

Asp Arg Phe Ser Ser Ser Gly Ser Gly Thr Asp Phe Thr Leu Arg Ile
65                  70                  75                  80

Ser Arg Val Glu Ala Glu Asp Val Gly Val Tyr Tyr Cys Ala Gln Asn
                85                  90                  95

Leu Glu Leu Pro Tyr Thr Phe Gly Gly Gly Thr Lys Leu Glu Ile Lys
            100                 105                 110

Arg

<210> 5
<211> 363
<212> DNA
<213> Mouse

<220>
<221> CDS
<222> (1)..(363)
<223>

<400> 5
cag gtt cag ctg cag cag tct gga gct gag ctg atg aag cct ggg gcc 48
Gln Val Gln Leu Gln Gln Ser Gly Ala Glu Leu Met Lys Pro Gly Ala
1               5                   10                  15

tca gtg aag ata tcc tgc aag gtt act ggc tac aca ttc agg agc tac 96
Ser Val Lys Ile Ser Cys Lys Val Thr Gly Tyr Thr Phe Arg Ser Tyr
            20                  25                  30

tgg ata gag tgg gta aag cag agg cct gga cat ggc ctt gag tgg att 144
Trp Ile Glu Trp Val Lys Gln Arg Pro Gly His Gly Leu Glu Trp Ile
        35                  40                  45

gga gag att tta gtt gga agt ggt agt act aag tat aat gag aag ttc 192
Gly Glu Ile Leu Val Gly Ser Gly Ser Thr Lys Tyr Asn Glu Lys Phe
    50                  55                  60

aag ggc aag gcc aca atc act gca cag aca tcc tcc aat aca gta tac 240
Lys Gly Lys Ala Thr Ile Thr Ala Gln Thr Ser Ser Asn Thr Val Tyr
65                  70                  75                  80

atg caa ctc agc agc ctg aca tct gag gac tct gcc gtc tat tac tgt 288
Met Gln Leu Ser Ser Leu Thr Ser Glu Asp Ser Ala Val Tyr Tyr Cys
                85                  90                  95

gca aga cct ttc ttc ggt agt agg tac gac tac tct gac ttc tgg ggc        336
Ala Arg Pro Phe Phe Gly Ser Arg Tyr Asp Tyr Ser Asp Phe Trp Gly
            100                 105                 110

caa ggc acc act ctc aca gtc tcc tca                                    363
Gln Gly Thr Thr Leu Thr Val Ser Ser
        115                 120

<210>  6
<211>  121
<212>  PRT
<213>  Mouse

<400>  6
Gln Val Gln Leu Gln Gln Ser Gly Ala Glu Leu Met Lys Pro Gly Ala
1               5                   10                  15

Ser Val Lys Ile Ser Cys Lys Val Thr Gly Tyr Thr Phe Arg Ser Tyr
            20                  25                  30

Trp Ile Glu Trp Val Lys Gln Arg Pro Gly His Gly Leu Glu Trp Ile
        35                  40                  45

Gly Glu Ile Leu Val Gly Ser Gly Ser Thr Lys Tyr Asn Glu Lys Phe
    50                  55                  60

Lys Gly Lys Ala Thr Ile Thr Ala Gln Thr Ser Ser Asn Thr Val Tyr
65                  70                  75                  80

Met Gln Leu Ser Ser Leu Thr Ser Glu Asp Ser Ala Val Tyr Tyr Cys
                85                  90                  95

Ala Arg Pro Phe Phe Gly Ser Arg Tyr Asp Tyr Ser Asp Phe Trp Gly
            100                 105                 110

Gln Gly Thr Thr Leu Thr Val Ser Ser
        115                 120

<210>  7
<211>  339
<212>  DNA
<213>  Mouse

<220>
<221>  CDS
<222>  (1)..(339)
<223>

<400>  7
gat att gtg atg acg cag gct gca ttc tcc aat cca gtc act ctt gga         48
Asp Ile Val Met Thr Gln Ala Ala Phe Ser Asn Pro Val Thr Leu Gly
1               5                   10                  15

tca tca gct tcc atc tcc tgc agg tct agt aag agt ctc cta cat aga         96
Ser Ser Ala Ser Ile Ser Cys Arg Ser Ser Lys Ser Leu Leu His Arg
            20                  25                  30

aat ggc atc act tat ttg tat tgg tat ctg cag aag cca ggc cag tct        144
Asn Gly Ile Thr Tyr Leu Tyr Trp Tyr Leu Gln Lys Pro Gly Gln Ser
        35                  40                  45

cct cag gtc ctg att tat cag atg tcc aac ctt gcc tca gga gtc cca        192

Pro Gln Val Leu Ile Tyr Gln Met Ser Asn Leu Ala Ser Gly Val Pro
    50                  55                  60

gac agg ttc agt agc agt ggg tca gga act gat ttc aca ctg aga atc      240
Asp Arg Phe Ser Ser Ser Gly Ser Gly Thr Asp Phe Thr Leu Arg Ile
65                  70                  75                  80

agc aga gtg gag gct gag gat gtg ggt gtt tat tac tgt gct caa aat      288
Ser Arg Val Glu Ala Glu Asp Val Gly Val Tyr Tyr Cys Ala Gln Asn
                85                  90                  95

cta gaa ctt ccg tac acg ttc gga ggg ggg acc aag ctg gaa ata aaa      336
Leu Glu Leu Pro Tyr Thr Phe Gly Gly Gly Thr Lys Leu Glu Ile Lys
            100                 105                 110

cgg                                                                  339
Arg

<210>  8
<211>  113
<212>  PRT
<213>  Mouse

<400>  8
Asp Ile Val Met Thr Gln Ala Ala Phe Ser Asn Pro Val Thr Leu Gly
1               5                   10                  15

Ser Ser Ala Ser Ile Ser Cys Arg Ser Ser Lys Ser Leu Leu His Arg
            20                  25                  30

Asn Gly Ile Thr Tyr Leu Tyr Trp Tyr Leu Gln Lys Pro Gly Gln Ser
        35                  40                  45

Pro Gln Val Leu Ile Tyr Gln Met Ser Asn Leu Ala Ser Gly Val Pro
    50                  55                  60

Asp Arg Phe Ser Ser Ser Gly Ser Gly Thr Asp Phe Thr Leu Arg Ile
65                  70                  75                  80

Ser Arg Val Glu Ala Glu Asp Val Gly Val Tyr Tyr Cys Ala Gln Asn
                85                  90                  95

Leu Glu Leu Pro Tyr Thr Phe Gly Gly Gly Thr Lys Leu Glu Ile Lys
            100                 105                 110

Arg

<210>  9
<211>  24
<212>  DNA
<213>  Artificial

<220>
<223>  Primer

<400>  9
gacagatggg ggtgtcgttt tggc                                            24

<210>  10
<211>  26
<212>  DNA
<213>  Artificial

<220>
<223>  Primer

<400>  10
ggaggaacca gttgtatctc cacacc                                          26

<210>  11
<211>  28
<212>  DNA
<213>  Artificial

<220>
<223>  Primer

<400>  11
agatggatac agttggtgca gcatcagc                                        28

<210>  12
<211>  29
<212>  DNA
<213>  Artificial

<220>
<223>  Primer

<400>  12
ccatggatca ggtccagctc cagcagtct                                       29

<210>  13
<211>  52
<212>  DNA
<213>  Artificial

<220>
<223>  Primer

<400>  13
agagccacct ccgcctgaac cgcctccacc tgaggagacg gtgactgagg tt             52

<210>  14
<211>  52
<212>  DNA
<213>  Artificial

<220>
<223>  Primer

<400>  14
ggcggaggtg gctctggcgg tggcggatcg gatattgtga tgacgcaggc tg             52

<210>  15
<211>  28
<212>  DNA
<213>  Artificial

<220>
<223>  Primer

<400>  15
gctagcccgt tttatttcca gcttggtc                                        28

<210>  16
<211>  29

<212>  DNA
<213>  Artificial

<220>
<223>  Primer

<400>  16
ccatggatca ggttcagctg cagcagtct                                          29

<210>  17
<211>  53
<212>  DNA
<213>  Artificial

<220>
<223>  Primer

<400>  17
agagccacct ccgcctgaac cgcctccacc tgaggagact gtgagagtgg tgc               53

<210>  18
<211>  30
<212>  DNA
<213>  Artificial

<220>
<223>  Primer

<400>  18
ccatggatga tattgtgatg acgcaggctg                                         30

<210>  19
<211>  52
<212>  DNA
<213>  Artificial

<220>
<223>  Primer

<400>  19
agagccacct ccgcctgaac cgcctccacc ccgttttatt tccagcttgg tc                52

<210>  20
<211>  51
<212>  DNA
<213>  Artificial

<220>
<223>  Primer

<400>  20
ggcggaggtg gctctggcgg tggcggatcg caggtccagc tccagcagtc t                 51

<210>  21
<211>  28
<212>  DNA
<213>  Artificial

<220>
<223>  Primer

<400>  21
gctagctgag gagacggtga ctgaggtt                                           28

<210>  22
<211>  51
<212>  DNA
<213>  Artificial

<220>
<223>  Primer

<400>  22
ggcggaggtg gctctggcgg tggcggatcg caggtccagc tccagcagtc t                51

<210>  23
<211>  29
<212>  DNA
<213>  Artificial

<220>
<223>  Primer

<400>  23
gctagctgag gagactgtga gagtggtgc                                         29

<210>  24
<211>  25
<212>  PRT
<213>  Artificial

<220>
<223>  Linker

<400>  24
Gly Gly Gly Gly Ser
1               5

<210>  25
<211>  14
<212>  PRT
<213>  Artificial

<220>
<223>  Linker

<400>  25
Gly Ser Thr Ser Gly Ser Gly Lys Ser Ser Glu Gly Lys Gly
1               5                   10

<210>  26
<211>  18
<212>  PRT
<213>  Artificial

<220>
<223>  Linker

<400>  26
Gly Ser Thr Ser Gly Ser Gly Lys Ser Ser Glu Gly Ser Gly Ser Thr
1               5                   10                  15

Lys Gly


<210>  27

<211>  12
<212>  PRT
<213>  Artificial

<220>
<223>  Linker

<400>  27
Gly Ser Thr Ser Gly Lys Pro Ser Glu Gly Lys Gly
1               5                   10

<210>  28
<211>  18
<212>  PRT
<213>  Artificial

<220>
<223>  Linker

<400>  28
Gly Ser Thr Ser Gly Ser Gly Lys Pro Gly Ser Gly Glu Gly Ser Thr
1               5                   10                  15

Lys Gly